# EUROPEAN PATENT SPECIFICATION

(11) **EP 2 687 998 B1**
(45) Date of publication and mention of the grant of the patent: **09.12.2015**
(21) Application number: 12758309.4
(22) Date of filing: 16.02.2012
(51) Int. Cl.: G06F 17/30

(54) **INFORMATION TERMINAL, INFORMATION PROVIDING SERVER, AND CONTROL PROGRAM**
INFORMATIONSENDGERÄT, INFORMATIONSBEREITSTELLUNGSSERVER UND STEUERPROGRAMM
TERMINAL D'INFORMATION, SERVEUR DE FOURNITURE D'INFORMATIONS, ET PROGRAMME DE COMMANDE

(30) Priority: 14.03.2011 JP 2011055554; 14.03.2011 JP 2011055555; 14.03.2011 JP 2011055847; 14.03.2011 JP 2011055848
(43) Date of publication of application: 22.01.2014
(73) Proprietor: Nikon Corporation, Tokyo 108-6290 (JP)
(72) Inventor: SUZUKI, Maki, Tokyo 100-8331 (JP); TAKE, Toshinori, Tokyo 100-8331 (JP); NAKADA, Yuko, Tokyo 100-8331 (JP); HOSOI, Kazuma, Tokyo 100-8331 (JP); UWAI, Hiroki, Tokyo 100-8331 (JP); SEKIGUCHI, Masakazu, Tokyo 100-8331 (JP)
(74) Representative: Hoffmann Eitle
(86) International application number: PCT/JP2012/001037
(87) International publication number: WO 2012/124259

(56) References cited:
- WO-A1-2004/061392
- WO-A1-2009/147711
- WO-A1-2010/032579
- JP-A- 10 105 602
- JP-A- 2002 351 954
- JP-A- 2003 157 393
- JP-A- 2004 127 063
- JP-A- 2004 206 401
- JP-A- 2008 242 837
- JP-A- 2008 267 741
- JP-A- 2009 116 552
- JP-A- 2009 271 853
- JP-A- 2010 072 818
- JP-A- 2011 039 862
- US-A1- 2006 156 209
- US-A1- 2008 125 959
- US-A1- 2010 131 443
- TOMOMASA SATO ET AL.: 'Environmental Robot as Start from Room Unit -sono Genjo to Shorai Tenkai' JOURNAL OF THE SOCIETY OF INSTRUMENT AND CONTROL ENGINEERS vol. 49, no. 6, 10 June 2010, pages 354 - 360, XP008171422
- HISASHI NISHIMAKI ET AL.: 'Behavior Recognition using an Image Sequence Captured from Different View Points for Healthcare of Ageing People' IEICE TECHNICAL REPORT(WIT2007-90-109) vol. 107, no. 555, 15 March 2008, pages 37 - 42, XP008170510
- HITOSHI KOSHIBA ET AL.: 'Blog Authoring Support System with Lifelog-aided Blog Template Generation' IPSJ JOURNAL vol. 51, no. 1, 15 January 2010, pages 63 - 81, XP008171030
- KATSUNORI MATSUOKA ET AL.: 'Personal Supporting System for Daily Life by Monitoring Human Behaviors in a House' SYSTEM/SEIGYO/JOHO vol. 46, no. 8, 15 August 2002, pages 32 - 37, XP055135940
- KIYOHARU AIZAWA: 'Ubiquitous Home ni Okeru Life Log no Shori to Kensaku' DATABASE TO WEB JOHO SYSTEM NI KANSURU SYMPOSIUM RONBUNSHU vol. 2006, no. 16, 30 November 2006, pages 9 - 12, XP008171779

## Description

### BACKGROUND

### 1. TECHNICAL FIELD

The present invention relates to an information terminal.

### 2. RELATED ART

A conventional portable information terminal has been proposed that assists a user, as shown in Japanese Patent Application Publication No. 2009-049950, for example.

Patent Document 1 proposes a technique for accumulating activity history of a user by having the portable information terminal with an image capturing function worn by the user automatically capture images periodically, to keep the experiences of the user as image data.

In US 2006/0156209 A1 there is described an application program prediction method and mobile terminal. The mobile terminal appropriately predicts an application that the user is likely to use. The mobile terminal includes an input device that selects and executes any of two or more applications, a GPS reception unit, a behavior pattern extraction unit that creates a usage prediction rule of the application executed on the input device, in association with a usage location detected by the GPS reception unit, and an information display control unit that specifies, based on the usage prediction rule, an application corresponding to the current location detected by the GPS reception unit, and causes a display to display the specified application as a prediction result.

However, a conventional portable terminal has trouble associating the gathered activity information with other information to create useful information to be supplied to the user.

### SUMMARY

According to a first aspect of the present invention, provided is an information terminal comprising the features of claim 1.

Preferred embodiments of the present invention are set forth in the dependent claims 2 to 10.

The summary clause does not necessarily describe all necessary features of the embodiments of the present invention. The present invention may also be a subcombination of the features described above.

### BRIEF DESCRIPTION OF THE DRAWINGS

Fig. 1 is a function block diagram of the personal assistance system.
Fig. 2 shows an exemplary person DB.
Fig. 3 shows exemplary sensor data.
Fig. 4 shows an exemplary activity DB.
Fig. 5 shows an example of activity information identified by the activity identifying section.
Fig. 6 is a flow chart showing a process of gathering activity information, performed by the information terminal.
Fig. 7 is a flow chart showing a process using the activity information stored in the storage section.
Fig. 8 is a flow chart showing a detailed first related information providing process.
Fig. 9 shows an exemplary schedule managed by the schedule managing section.
Fig. 10 is a flow chart showing a process using the activity information according to the second embodiment.
Fig. 11 shows an exemplary activity prediction table for luggage information.
Fig. 12 shows an exemplary activity prediction table for companions.
Fig. 13 shows an exemplary activity prediction table for weather.
Fig. 14 is a flow chart showing a detailed information providing process for the schedule.
Fig. 15 is a flow chart showing a detailed related information displaying process.

### DESCRIPTION OF EXEMPLARY EMBODIMENTS

Hereinafter, some embodiments of the present invention will be described. The embodiments do not limit the invention according to the claims, and all the combinations of the features described in the embodiments are not necessarily essential to means provided by aspects of the invention.

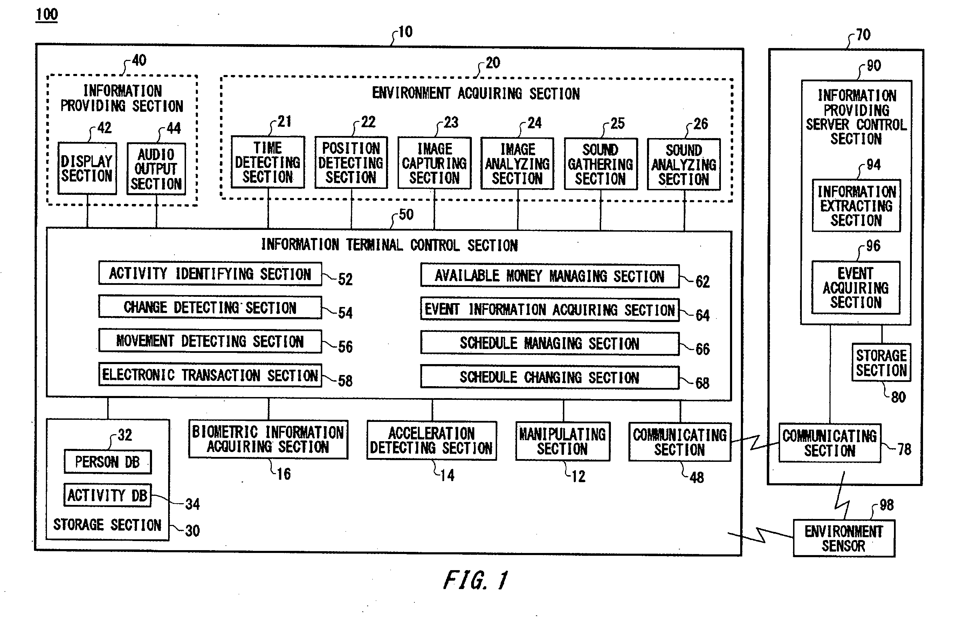

Fig. 1 is a function block diagram showing the system configuration of a personal assistance system 100 according to an embodiment of the present invention. As shown in Fig. 1, the personal assistance system 100 includes an information terminal 10 and an information providing server 70.

The information terminal 10 is a terminal that can be carried by a user, and may be a mobile phone, a smart phone, a PHS (Personal Handy-phone System), a PDA (Personal Digital Assistant), or the like. The size of the information terminal 10 may be such that the information terminal 10 can be inserted into a pocket. The user carries the information terminal 10 by clipping the information terminal 10 to clothing or hanging the information terminal 10 from the neck. As shown in Fig. 1, the information terminal 10 includes a manipulating section 12, an acceleration detecting section 14, a biometric information acquiring section 16, an environment acquiring section 20, a storage section 30, an information providing section 40, and an information terminal control section 50.

The manipulating section 12 includes an input interface such as a keyboard or touch panel. The acceleration detecting section 14 is an acceleration sensor, and detects acceleration of the information terminal 10.

The biometric information acquiring section 16 acquires biometric information of the user. The biometric information acquiring section 16 acquires at least one type of biometric information such as the muscle state (nervous or relaxed), blood pressure, heart rate, pulse, amount of sweat, and body temperature of the user, for example. The method for acquiring the biometric information may involve adopting a wrist-watch device such as described in Japanese Patent Application Publication No. 2005-270543 or Japanese Patent Application Publication No. 2007-215749 (US Patent Application Publication No. 20070191718). In this case, the structure is separate from the information terminal 10, and therefore the information terminal 10 receives output results with the biometric information acquiring section 16. Furthermore, the blood pressure and the pulse may be detected by a pulse wave sensor using infrared rays, and the heart rate may be detected by a vibration sensor. In the present embodiment, the data measured by a weight scale or body fat detecting scale in the home of the user is output to the information terminal 10 wirelessly or through operation of a manipulating section 12.

In the manner described above, the biometric information acquiring section 16 is formed by combining a variety of sensors, and each sensor outputs a different type of biometric information. These outputs can be analyzed individually or in combination to estimate a prescribed emotion of the user. For example, when a high heart rate and emotional sweat are detected, it can be estimated that the photographer is feeling "rushed." The relationship between the output of the sensors and the emotion is obtained verifiably, and can be stored in advance in a storage section 30 as a table showing the corresponding relationship. When estimating the emotion, a judgment may be made as to whether the acquired biometric information matches a prescribed emotion pattern recorded in the table.

The environment acquiring section 20 includes a time detecting section 21, a position detecting section 22, an image capturing section 23, an image analyzing section 24, a sound gathering section 25, and a sound analyzing section 26. The time detecting section 21 has a clock function to detect the current time. The position detecting section 22 detects the position of the information terminal 10. The position detecting section 22 includes a GPS (Global Positioning System), for example.

The image capturing section 23 includes an image capturing sensor such as a CCD or CMOS, and captures images of at least a portion of the environment around the information terminal 10. The image analyzing section 24 may be an image processing chip such as an ASIC (Application Specific Integrated Circuit), for example, and analyzes the images captured by the image capturing section 23. The image analyzing section 24 identifies subjects such as people included in the image, by performing pattern recognition using image feature amounts registered in advance in an image database, for example. The image database may be included in the information terminal 10, or may be included in an external server. The sound gathering section 25 is a microphone, for example, and gathers at least a portion of the sound in the environment around the information terminal 10. The sound analyzing section 26 is a sound processing chip, for example, and analyzes the sound gathered by the sound gathering section 25. The sound analyzing section 26 performs speaker identification by performing a voice analysis and converts sound into text through voice recognition, for example. The voice analysis identifies the speaker by using sound feature characteristics such as the size of the voice (loudness), frequency of the voice, or length of the sound to perform pattern matching with registered voice data.

The storage section 30 includes a non-volatile storage device such as a hard disk and flash memory, and stores the various types of data processed by the information terminal 10. The information providing section 40 includes a display section 42 having a display control section and an audio output section 44 having a sound output section. The display section 42 includes an LCD panel, for example, displays an image, text, and the like, and also displays a menu enabling the user to perform manipulations. The audio output section 44 includes a speaker and outputs sound and voice.

The communicating section 48 includes a wireless communication unit that accesses a wide area network such as the Internet, a Bluetooth (registered trademark) unit that realizes communication via Bluetooth (registered trademark), and an IC chip such as a Felica (registered trademark) chip. In the present embodiment, the information terminal 10 can realize communication with another information terminal, an information providing server 70, and an environment sensor 98, via the communicating section 48.

The information terminal control section 50 includes a CPU, for example, and performs overall control of each component in the information terminal 10 to perform processing in the information terminal 10. Here, the data acquired by the information terminal control section 50 via the acceleration detecting section 14, the biometric information acquiring section 16, and the environment acquiring section 20 is referred to as "sensor data." The sensor data acquired by the information terminal control section 50 is stored in the storage section 30. The information terminal control section 50 includes an activity identifying section 52, a change detecting section 54, a movement detecting section 56, an electronic transaction section 58, an available money managing section 62, an event information acquiring section 64, a schedule managing section 66, and a schedule changing section 68.

The activity identifying section 52 identifies activity information of the user by referencing the acquired sensor data with the correspondence database indicating a relationship between the activity and the sensor data. The activity information of the user indicates what type of activity the user is doing. The correspondence database indicating the relationship between the activity and the sensor data may be basic information stored in the storage section 30 at the time when the information terminal 10 is manufactured. Content specific to the user may be stored in the storage section 30 by using the manipulating section 12, or stored in the storage section 30 through sound input using the sound gathering section 25. In the present invention, an activity DB 34 is stored in the storage section 30 as the correspondence database. The activity identifying section 52 accumulates specified activity information in the storage section 30. The activity identifying section 52 may identify the activity of the user from the acquired sensor data, without referencing the activity DB 34.

The change detecting section 54 detects change in the activity information of the user identified by the activity identifying section 52. For example, the change detecting section 54 may detect, as change in the activity information, the user beginning the identified activity at a set time every day. As another example, the change detecting section 54 may detect, as change in the activity information, a change in the content of the activity performed at a predetermined time. When the frequency of an activity corresponding to the detected change exceeds a predetermined frequency, the change detecting section 54 judges this activity to be a habit of the user. The change detecting section 54 accumulates in the storage section 30, as habit data indicating habits, the activity information for activities judged to be habits. The specific method for detecting a habit is described further below.

The movement detecting section 56 detects movement of the user from the detection data of at least one of the position detecting section 22 and the acceleration detecting section 14. For example, the movement detecting section 56 may continuously acquire position data detected by the position detecting section 22 and detect the movement speed of the user based on change per unit time in the position data. As another example, the movement detecting section 56 may integrate the acceleration detected by the acceleration detecting section 14 and use the result as support in addition to the detection data of the position detecting section 22 to detect the movement speed of the user. In addition, a gyro that is an angular velocity sensor may be provided in a shoe or in the wrist-watch described above, in order to calculate the speed from the linked motion of the arms and the feet when walking or jogging. In the present embodiment, based on the integral value of the acceleration detected by the acceleration detecting section 14 and the movement detecting section 56, the normal walking speed of the user is detected to be 4 to 5 Km/hour, the power walking (walking for exercise) speed of the user is detected to be 5 to 7 Km/hour, and the jogging speed of the user is detected to be 8 to 11 Km/hour.

The electronic transaction section 58 performs an electronic transaction via the IC chip of the communicating section 48. For example, the electronic transaction section 58 may purchase a drink such as a can of juice by communicating with a vending machine located nearby. Completion of this purchase may be realized using electronic money stored in the storage section 30 or by using a credit card through a transaction server. If the purchase is made by a credit card through a transaction server, the electronic transaction section 58 can exchange information concerning the purchased products by referencing the purchase history recorded in the transaction server via the communicating section 48. The available money managing section 62 manages the available money of the user. In the present embodiment, the available money managing section 62 manages electronic money stored in the storage section 30, as the available money of the user.

The event information acquiring section 64 acquires event information relating to the activity of the user. The event information acquiring section 64 acquires the event information relating to the activity of the user by referencing association data that associates the activity information with event information. For example, if the association data associates the activity of driving a car with traffic information, the event information acquiring section 64 acquires, as the event information, traffic information for the activity information of driving a car. The event information acquiring section 64 may reference association data stored in the storage section 30, or may reference association data stored in the storage section 80 of the information providing server 70 via the communicating section 48.

The schedule managing section 66 manages a schedule of the user that is input by the user via the manipulating section 12 and stored in the storage section 30. The schedule changing section 68 changes the schedule of the user stored in the storage section 30. For example, the schedule changing section 68 may add, to the schedule of the user stored in the storage section 30, a habit of the user detected by the change detecting section 54.

The information providing server 70 is a server connected to a network such as a high-speed LAN and the Internet. As shown in Fig. 1, the information providing server 70 includes a communicating section 78, a storage section 80, and an information providing server control section 90.

The communicating section 78 has the same configuration as the communicating section 48 of the information terminal 10. The information providing server 70 can communicate with the communicating section 48 of the information terminal 10 via the communicating section 78. For example, the information providing server 70 receives the activity information of the user of the information terminal 10 from the information terminal 10. Furthermore, the information providing server 70 receives the habit data of the user of the information terminal 10 from the information terminal 10.

The storage section 80 includes a non-volatile storage device such as a hard disk and a flash memory, and accumulates the habit data and activity information of the user of the information terminal 10 received via the communicating section 78. A management ID for identifying the information terminal 10 is allocated in advance to the information terminal 10, and the information terminal 10 transmits the allocated management ID to the information providing server 70 along with the habit data and the activity information of the user. The storage section 80 accumulates the habit data and activity information received by the communicating section 78 in an accumulation region corresponding to the received management ID.

The information providing server control section 90 includes a CPU, for example, and performs overall control of each component in the information providing server 70 to perform processing in the information providing server 70. The information providing server control section 90 includes an information extracting section 94 and an event information acquiring section 96.

The information extracting section 94 extracts from the storage section 80 information relating to the activity corresponding to the habit data received from the information terminal 10. For example, the information extracting section 94 extracts from the storage section 80 a webpage relating to the activity, an image relating to the activity, and the like. The information extracted by the information extracting section 94 is transmitted to the information terminal 10 by the information providing server control section 90, via the communicating section 78. The event information acquiring section 96 acquires from the storage section 80 event information related to the activity information of the user of the information terminal 10 received from the information terminal 10 via the communicating section 78.

The environment sensor 98 is a sensor arranged near the information terminal 10. For example, the environment sensor 98 may be a webcam arranged in a meeting room to capture images of a meeting or arranged on the side of a road to capture images of the road. The environment sensor 98 has a communication function and can communicate with the information terminal 10. For example, the meeting room camera captures images of the meeting room and images of people in the meeting room including the user of the information terminal 10, and transmits the captured images to the information terminal 10 held by the user. The communication between the meeting room camera and the information terminal 10 held by the user can be realized through Bluetooth (registered trademark), for example.

The information terminal 10 can acquire from the environment sensor 98 information relating to the environment around the information terminal 10, which may be images of the area around the information terminal 10 and images of people in this area, via the communicating section 48. The information terminal control section 50 accumulates in the storage section 30, as the sensor data, the acquired information relating to the surrounding environment. In this way, the information terminal 10 collects the activity information of the user not only from the components of the information terminal 10, but also from the environment sensor 98. Therefore, information that can be difficult to acquire using a sensor of the information terminal 10, such as an image of the user, can be acquired easily.

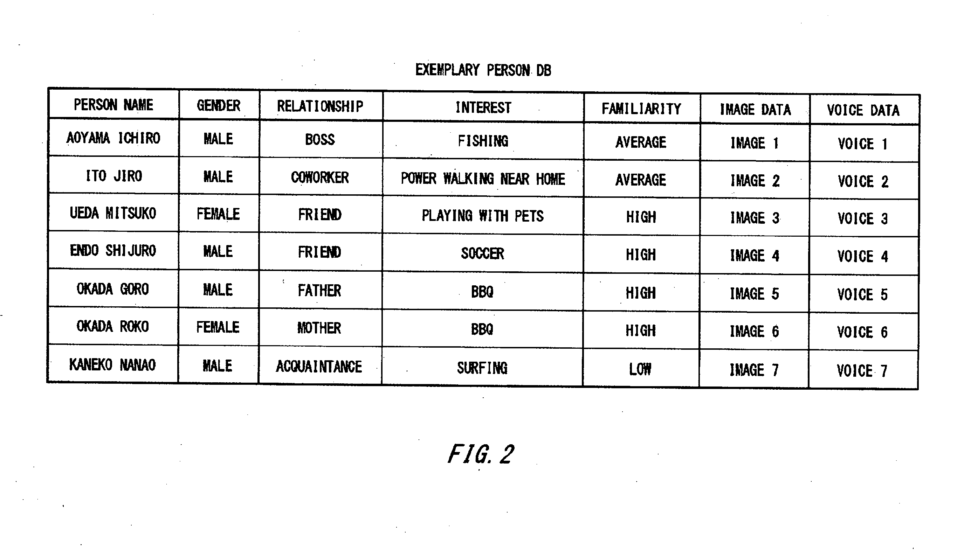

Fig. 2 shows an example of a person DB 32, which is a database in which people are registered in advance by the user. In the person DB 32, personal information of each person is registered in association with the name of the person. The personal information is information about the person, and in the example of Fig. 2, the gender, relationship to the user, hobbies, and the familiarity to the user are registered for each person. Furthermore, information identifying image data in which the person is captured and information identifying voice data of the person are also stored in the person DB 32 for each person. The image data and the voice data are stored in the storage section 30, and pointers for each piece of image data and each piece of voice data are registered in the person DB 32. For example, "image 1" is a pointer identifying the image data in which "Aoyama Ichiro" is captured.

The pointer identifying the image data registered in the person DB 32 may be used when the image analyzing section 24 identifies a person included in an image captured by the image capturing section 23, for example. The image analyzing section 24 identifies image data that satisfies a resemblance condition, by comparing the image data of the image captured by the image capturing section 23 to a plurality of pieces of image data stored in the storage section 30. When the pointer identifying image data that fulfills the resemblance condition is registered in the person DB 32, the image analyzing section 24 identifies a person. In the same manner, a pointer identifying voice data registered in the person DB 32 is used when the sound analyzing section 26 identifies a person whose voice has been gathered by the sound gathering section 25, for example.

If a person cannot be identified from the output of one of the image analyzing section 24 and the sound analyzing section 26, the information terminal control section 50 may reference the output of the other of the image analyzing section 24 and the sound analyzing section 26 to identify the person. In addition, the information terminal control section 50 may identify the person by referencing the time detected by the time detecting section 21 or the location detected by the position detecting section 22. In this way, the accuracy of identifying a person is increased.

The familiarity indicates the level of familiarity between the user and each person. In the example of Fig. 2, a high familiarity is set for parents and friends, and a low familiarity is set for Kaneko Nanao, who is merely an acquaintance. This embodiment describes an example in which the information terminal control section 50 stores personal information input by the user in the storage section 30, but instead, if the information terminal 10 has a function to send and receive mail, the information terminal control section 50 may reference sent and received mail data and address book data to register familiarity levels. Specifically, the information terminal control section 50 references the mail sent to and received from a person registered in the person DB 32, and registers a high or low familiarity in the person DB 32 according to whether the frequency of sending and receiving mail to and from this person is high or low.

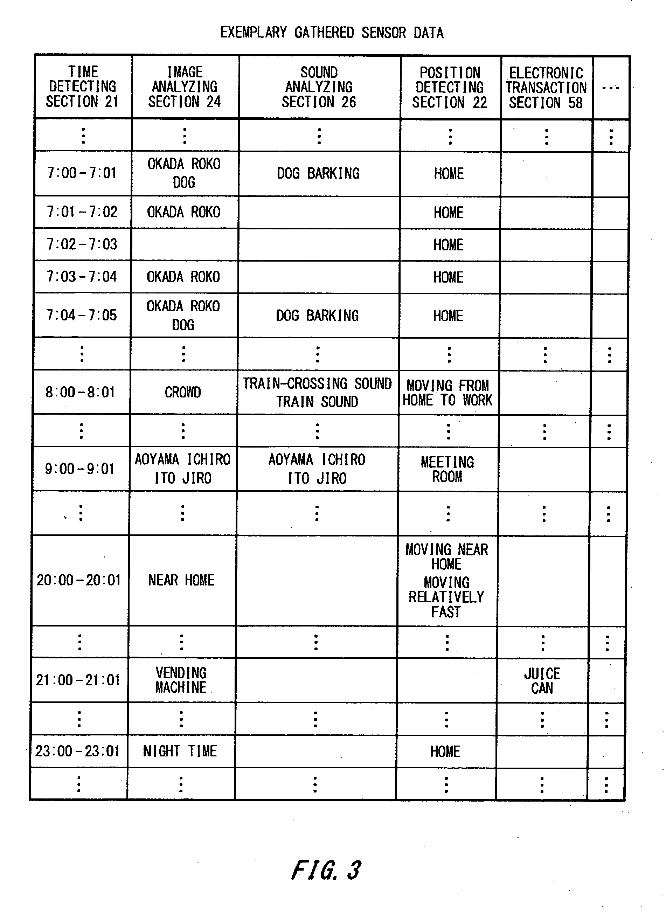

Fig. 3 shows an example of sensor data acquired by the information terminal 10. The leftmost column in the table of Fig. 3 shows the time span during which the sensor data was acquired by the time detecting section 21. The "image" column, which is adjacent to the column showing the time spans, shows results obtained by the image analyzing section 24 analyzing the image captured by the image capturing section 23 during the corresponding time span. The "sound" column, which is adjacent to the "image" column, shows results obtained by the sound analyzing section 26 analyzing the sound gathered by the sound gathering section 25. The "position information" column, which is adjacent to the "sound" column, shows position information detected by the position detecting section 22. The "purchase information" column, which is adjacent to the "position information" column, shows purchase information acquired by the electronic transaction section 58.

In the example of Fig. 3, images of "Okada Roko" and a "dog" are captured by the image capturing section 23 of the user from 7:00 to 7:01. As described above, by using the person DB 32, the image analyzing section 24 identifies "Okada Roko" as a person included in the image captured by the image capturing section 23. Furthermore, the image analyzing section 24 can identify the subjects by referencing an image recognition database stored in advance. The image recognition database has a plurality of patterns, such as an image of a dog and an image of a vending machine, registered therein, and the image analyzing section 24 identifies the subjects by performing pattern recognition between the captured image and images registered in the image recognition database. The image recognition database may be stored in the storage section 30, or an image recognition database stored in the storage section 30 of the information providing server may be referenced.

When a person registered in the person DB 32 is recognized, the image analyzing section 24 can detect the surrounding environment of the information terminal 10 by referencing the relationship with this person. For example, when the person identified by the image analyzing section 24 is a "father," "mother," or "friend," a private situation is acquired as the surrounding environment, and when the person identified by the image analyzing section 24 is a "boss" or "co-worker," a non-private situation is acquired as the surrounding environment. In the manner described above, when the image analyzing section 24 identifies "Okada Roko," since the relationship of "Okada Roko" to the user of the information terminal 10 is determined to be "mother" by referencing the person DB 32, a private situation is acquired as the surrounding environment. The relationships in the person DB 32 are associated with private situations and non-private situations in advance.

"Barking of a dog" is acquired as a sound. The sound analyzing section 26 identifies the barking of a dog by referencing a sound recognition database stored in the storage section 80 of the information providing server 70 or the storage section 30. "Home" is acquired as position information. The position detecting section 22 identifies the name of the location indicated by the position information based on the position data detected by the GPS, for example, by referencing map data stored in the storage section 30. The map data stored in the storage section 30 includes data associating the name of locations included in the map with the position data corresponding to this location. The position data corresponding to the location of the home in the map is recorded in advance by the user. The position detecting section 22 may reference the map data stored in the storage section 80 of the information providing server via the communicating section 48.

Movement information can be acquired as the position information. In the example of Fig. 3, the information "movement near home" and "relatively fast movement" is acquired from 20:00 to 20:01. The information terminal control section 50 determines "movement near home" in a case where the position detected by the position detecting section 22 is within a prescribed range of "home" and movement is detected by the movement detecting section 56. In this case, it is possible that the user is power walking or quickly returning home. The information terminal control section 50 may judge whether the user is power walking or returning home (movement between the closest train station and home) based on the output of the position detecting section 22. Instead, the information terminal control section 50 may detect, via the time detecting section 21, the time during which the user is moving quickly. Specifically, it is expected that the user needs approximately 15 minutes to return home when walking quickly and that the user continues for at least 30 minutes when power walking. Therefore, the information terminal control section 50 can determine whether the user is power walking or returning home based on the time during which the user moves quickly.

When the movement speed detected by the movement detecting section 56 is less than a predetermined movement speed, the information terminal control section 50 determines "slow movement." When "slow movement" is determined, situations such as the user carrying heavy luggage, the user walking with a child, or the user feeling unwell can be imagined. The information terminal control section 50 determines which of these cases is occurring based on the outputs of the environment acquiring section 20, the biometric information acquiring section 16, and the like.

The position detecting section 22 may acquire the position information from the environment sensor 98, by communicating with the environment sensor 98 via the communicating section 48. For example, when the user holding the information terminal 10 is in a company meeting room and the information terminal 10 is performing short-range communication with a meeting room camera arranged in the meeting room, the position detecting section 22 can receive the position information indicating the "meeting room" from the meeting room camera.

If an authentication system is introduced that authenticates people entering and exiting the meeting room, the information terminal 10 can acquire position information by communicating with the authentication system. For example, for an authentication system in which the authentication ID for authenticating a person entering or exiting the meeting room is stored in the storage section 30 and the information terminal 10 is swiped over an authentication reader arranged at the entrance of the meeting room in order to enter, the entrance of the user of the information terminal 10 into the meeting room is registered. The information terminal 10 can acquire the "meeting room" as the position information, by acquiring from the authentication system the entrance registration corresponding to the authentication ID of the storage section 30.

The purchase information shows information concerning items purchased by the user using the information terminal 10. The information terminal control section 50 acquires the purchase information via the electronic transaction section 58 and the time detecting section 21. In the example of Fig. 3, a "juice can" is registered as the purchase information from 21:00 to 21:01.

Fig. 3 describes an example in which analysis results of the acquired data are registered every other minute, but the time interval between registrations is not limited to this. Instead, the analysis results can be acquired when there is a change in the feature amount of the acquired data. For example, the information terminal control section 50 may have the image analyzing section 24 continually analyze the image feature amount of the image captured by the image capturing section 23. When the change in the image feature amount exceeds a predetermined value, the information terminal control section 50 may determine that the feature amount of the acquired data has changed.

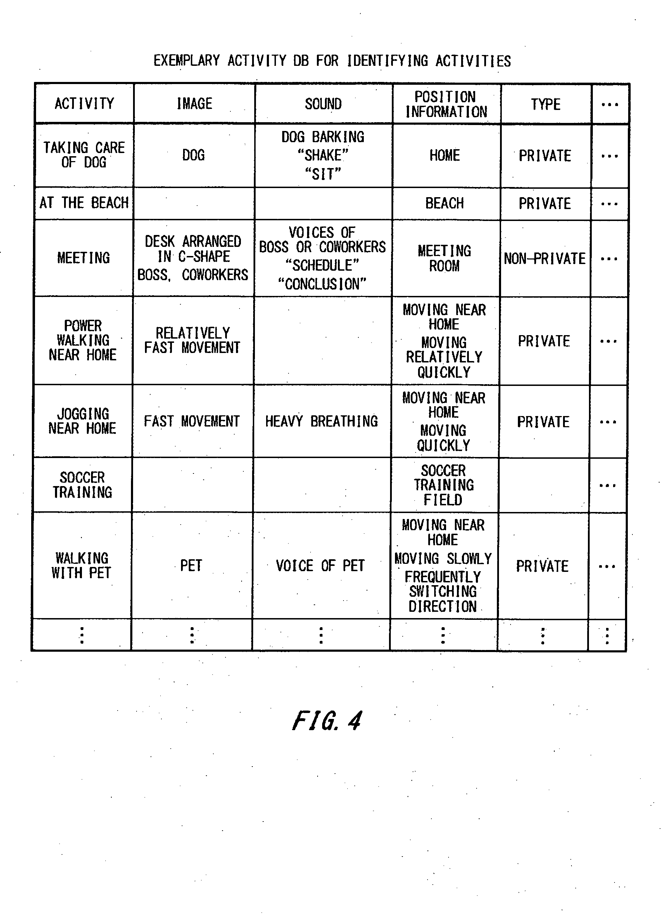

Fig. 4 shows an example of the activity DB 34 stored in the storage section 30. The activity DB 34 is a correspondence database indicating the relationship between the activities and sensor data, as described above. Activities such as "being at the beach," "taking care of the dog," and "power walking near home" are registered in the activity DB 34 in association with sensor data expected to be detected when the corresponding activity is performed. The sensor data associated with the activities registered in the activity DB 34 is referred to as "activity identification conditions." In Fig. 4, images, sound, and position information are registered as examples of the activity identification conditions. Furthermore, classification data, which indicates whether each activity is a private activity or a non-private activity, is registered in the activity DB 34.

When position data corresponding to the beach is acquired from the position detecting section 22 and the time spent at the beach detected by the time detecting section 21 exceeds a predetermined time, e.g. 10 minutes, the activity identifying section 52 identifies the activity as "being at the beach." When the image of a dog and the sound of a dog barking are acquired within a predetermined time, e.g. 1 minute, and the position information indicates the home, for example, the activity identifying section 52 identifies the activity as "taking care of the dog." In other words, the activity identifying section 52 can identify the activity by using not only one type of sensor data, but by matching a plurality of types of sensor data to the activity identification conditions of the activity DB 34.

Keywords included in voices gathered by the sound gathering section 25 in the manner described above may be registered in the activity DB 34 as activity identification conditions. For example, as a condition for identifying taking care of the dog, keywords relating to dog training such as "shake" and "sit" can be registered. Information of a person identified from the sensor data may also be used as an activity identification condition for indentifying an activity. For example, when the position information indicates a meeting room and an image of a boss or coworker is continuously captured, the activity of "meeting" can be identified.

In Fig. 4, a plurality of activity identification conditions are registered for the activity "meeting," and the activity identifying section 52 may identify the activity when all of the activity conditions are met or may identify the activity when predetermined activity identification conditions among the plurality of activity identification conditions are met. For example, the activity identifying section 52 can identify the activity of "meeting" when any one of the conditions of the position information indicating a meeting room, the voice of a boss or coworker being detected, a keyword of "schedule" being detected, or a keyword of "conclusion" being detected is fulfilled. The conditions for identifying an activity may be obtained by the information terminal control section 50 storing conditions input by the user via the manipulating section 12 in the storage section 30.

The activity identifying section 52 may identify an activity based on the movement of the user detected by the movement detecting section 56. For example, when the activity of being at the beach is detected, if output from the acceleration detecting section 14 is not detected, there is a high possibility that the information terminal 10 is not being worn and has been left in a locker, for example, and therefore an activity that takes place at the beach and requires removing the information terminal 10, such as surfing or swimming, can be identified.

When output from the acceleration detecting section 14 is detected, the user is moving on the beach, and therefore the activity identifying section 52 can identify an activity that can be done at the beach while wearing the information terminal 10, such as fishing or a BBQ. When the movement detecting section 56 detects movement of a distance greater than a predetermined distance, such as the distance from home to the nearest train station, if the movement speed is greater than the normal walking speed, the activity identifying section 52 may identify an activity of jogging on the beach. In this way, by identifying an activity based on the movement of the user, the activity of the user can be more accurately identified. If the acquired sensor data fulfills the activity identification conditions of a plurality of activities, in the present embodiment, whichever activity is identified first is used.

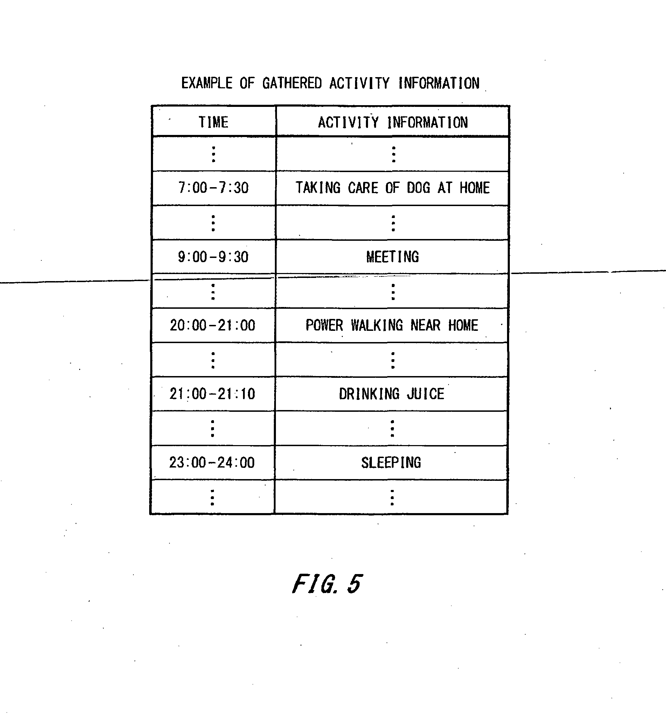

Fig. 5 shows an example of activity information gathered and identified by the activity identifying section 52. Fig. 5 shows an example of activity information gathered from the sensor data shown in Fig. 3. When the acquired sensor data fulfills activity identification conditions registered in the activity DB 34, the activity identifying section 52 identifies the activity registered in association with the fulfilled activity identification conditions as the activity being performed. Furthermore, if the sensor data still fulfills the same activity identification conditions with a certain time, e.g. 5 minutes, after identifying the activity, the activity identifying section 52 determines that this activity is continuing.

In the case of the sensor data in Fig. 3, for example, an image of a dog and a sound of a dog barking are acquired and the position information indicates home, from 7:00 to 7:01, and therefore the activity identifying section 52 identifies "taking care of the dog at home" as the activity information. This activity identification condition is not fulfilled from 7:01 to 7:03, but is fulfilled from 7:04 to 7:05, and therefore the activity identifying section 52 determines that the activity of taking care of the dog is continuing. When it is determined that the identified activity is continuing, the activity identifying section 52 determines whether the same activity identification condition is still fulfilled within 5 minutes from the determination. If the same activity identification condition is not fulfilled for 5 minutes or more, the activity identifying section 52 determines that this activity has ended. In this example, the activity of taking care of the dog continues until 7:30.

From 20:00 to 21:00, the activity identifying section 52 identifies "power walking near home" as the activity information, based on the sensor data of "movement near home" and "relatively fast movement" from 20:00 to 20:01 shown in Fig. 3 fulfilling the activity identification condition of "power walking near home" in the activity DB 34. As described above, the activity identifying section 52 may identify the activity of power walking near home based on the sensor data without referencing the activity DB 34. In this example, the activity of power walking near home lasts until 21:00. From 23:00 until 24:00, the image is black and the position information indicates the home, and therefore the activity identifying section 52 identifies the activity of "sleeping." The activity identifying section 52 may identify "sleeping" by communicating with a pressure sensor spread under the bed.

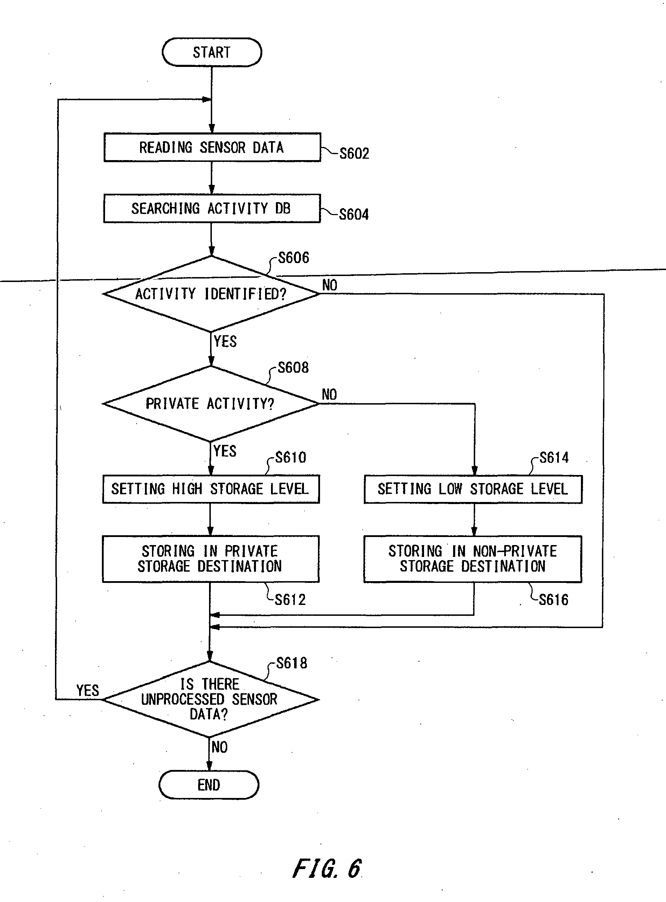

Fig. 6 is a flow chart of the process for collecting activity information performed by the information terminal 10. The information terminal 10 performs the activity information gathering process shown in this flow chart periodically, e.g. every 10 minutes. First, at step S602, the activity identifying section 52 reads the sensor data stored in the storage section 30.

At step S604, the activity identifying section 52 identifies the activity by searching the activity DB 34 based on the read sensor data. At step S606, the information terminal control section 50 determines whether an activity was able to be identified by the activity identifying section 52. If the result of the search of the activity DB 34 is that there is no matching activity, the information terminal control section 50 determines that an activity could not be identified. If an activity is identified, the process moves to step S608, and if an activity could not be identified, the process moves to step S618.

At step S608, the information terminal control section 50 determines whether the activity identified at step S606 is a private activity. The information terminal control section 50 determines whether the activity is private by referencing the classification column for the identified activity in the activity DB 34. If the identified activity is private, the process proceeds to step S610.

At step S610, the activity identifying section 52 sets the storage level for storing the activity information to be high. The storage level is a value indicating the amount of detail in the information when storing the acquired sensor data. For example, when the storage level is set to be low, the image captured by the image capturing section 23 is stored with lower resolution than when the storage level is set to be high. By storing the image with a lower resolution in this way, when the image capturing section 23 captures an image of a white board at a meeting, for example, the image is stored with a resolution that makes it impossible to read the characters on the white board, and therefore leaking of confidential information can be prevented. Instead of lowering the image resolution, the information terminal control section 50 may prohibit image capturing by the image capturing section 23. Furthermore, the information terminal control section 50 may display map information in the display section 42 and designate a business region that is a non-private region for the user, and may lower the image capturing resolution of the image capturing section 23 or prohibit image capturing by the image capturing section 23 when the position detecting section 22 detects the business region.

At step S612, the activity identifying section 52 stores the identified activity information at the private relationship storage destination. The private relationship storage destination may be an information providing server 70 that is arranged in a public network such as the Internet. The information terminal control section 50 transmits the activity information to the information providing server 70 through the communicating section 48, along with the management ID allocated to the information providing server 70. The information providing server 70 registers the received activity information in the accumulation region of the storage section 80 matching the received management ID.

On the other hand, if it is determined at step S608 that the activity is not a private activity, the process moves to step S614. At step S614, the activity identifying section 52 sets the storage level to be low. By setting the storage level to be low, the acquired sensor data is registered with a low amount of detail. Instead, the information terminal control section 50 may prohibit the storage of the sensor data or prohibit the acquisition of environment information by the environment acquiring section 20.

At step S616, the activity identifying section 52 stores the activity information in the non-private relationship storage destination. The non-private relationship storage destination may be an information providing server 70 arranged in a company that can be accessed from within the company, for example. By registering the non-private activity information in a storage destination with a high security level, such as at a company, leaking of confidential information can be prevented.

At step S618, the information terminal control section 50 determines whether there is censor data that has yet to be processed. If it is determined that there is unprocessed sensor data, the process moves to step S602 and the unprocessed sensor data is read. If it is determined that there is no unprocessed sensor data, the process is finished. The flow chart shown in Fig. 6 shows an example in which both change of the storage level and change of the storage destination are performed according to whether the activity is a private activity, but instead, just one of change of the storage level and change of the storage destination may be performed.

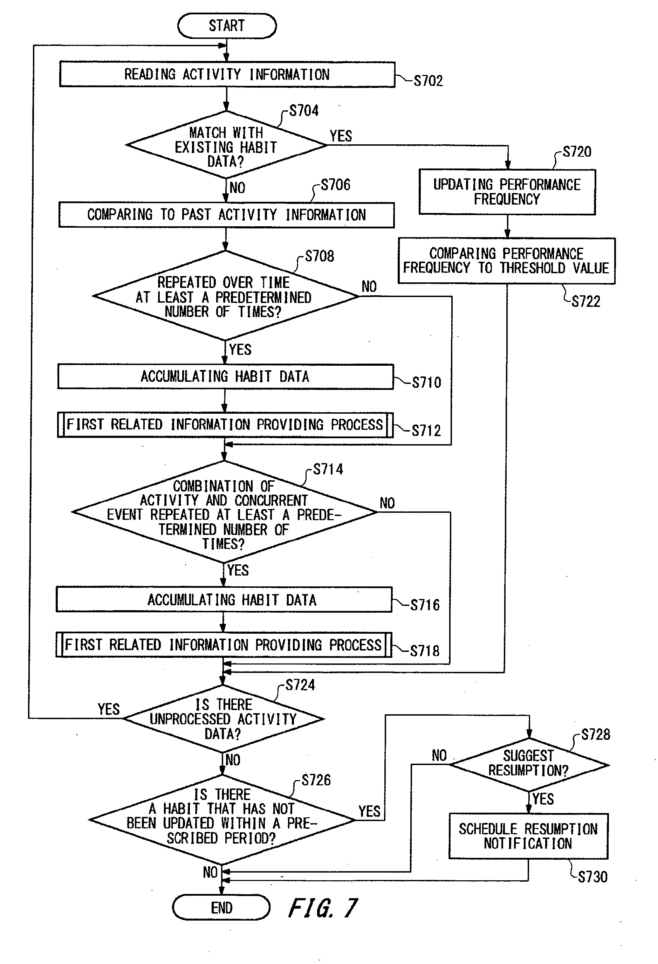

Fig. 7 is a flow chart showing a process of using the activity information stored in the storage section 30 according to the first embodiment. The present embodiment describes an example in which the information terminal 10 performs this process flow at 12:00 every night.

At step S702, the information terminal control section 50 reads from the storage section 30 one piece of activity information registered during the day. At step S704 the change detecting section 54 determines whether the read activity information matches existing habit data accumulated in the storage section 30. For example, when the read activity information is "power walking near home from 20:00 to 21:00" and the habit data "power walking near home from 20:00 to 21:00" is accumulated in the storage section 30, it is determined that this activity information matches an existing habit. If the activity information does not match an existing habit, the process moves to step S706.

At step S706, the change detecting section 54 compares the read activity information to past activity information already accumulated in the storage section 30. At step S708, the information terminal control section 50 determines whether the activity indicated by the read activity information has been repeated a predetermined number of times over time.

The change detecting section 54 determines whether the activity has been repeated during each of a plurality of different periods. For example, the change detecting section 54 determines whether the activity has been repeated every day, every two days, every three days, etc. or every week, every two weeks, every three weeks, etc. Furthermore, the change detecting section 54 determines whether there is a particular pattern to the repetition. For example, the change detecting section 54 determines if the activity is repeated on the same day or if the activity is repeated every holiday.

If repetition is detected, the change detecting section 54 determines whether the number of repetitions is greater than or equal to a predetermined number of times. The predetermined number of times is stored in the storage section 30 in advance, and may be a number such as three times or five times. A different predetermined number of times may be set for each period. For example, the number of times an activity was repeated during each period may be set to be five times if repeated every day or may be three times if repeated every month. The predetermined number of times can be changed according to input by the user through the manipulating section 12. The process moves to step S710 when the information terminal control section 50 determines at S708 that the activity has been repeated at least the predetermined number of times, and the process moves to step S714 when the information terminal control section 50 determines at S708 that the activity has not been repeated at least the predetermined number of times.

At step S710, the information terminal control section 50 accumulates the read activity information in the storage section 30, as new habit data. When a deletion instruction for deleting habit accumulation is received from the user through the manipulating section 12, the information terminal control section 50 deletes the habit data stored in the storage section 30 corresponding to the habit indicated in the deletion instruction. The information terminal control section 50 displays a list of existing habit data stored in the storage section 30 by controlling the display section 42. The information terminal control section 50 receives the deletion instruction by receiving, through the manipulating section 12, a selecting instruction of the user with respect to the displayed list.

At step S712, the information providing section 40 performs a first related information providing process for providing information related to the activities accumulated as habits, in response to instructions from the information terminal control section 50. For example, when a habit of power walking near home is newly detected, the information providing section 40 provides, as information related to power walking, a recommended power walking course or a webpage selling power walking shoes, for example. The first related information providing process is described in detail further below.

At step S714, the change detecting section 54 determines whether a combination of the activity indicated by the read activity information and a concurrent event are repeated at least a predetermined number of times. Here, a "concurrent event" refers to an activity performed along with another activity. For example, if juice is drunk after power walking, then the activity of drinking juice is a concurrent event for power walking, and if a restaurant is visited after soccer training, then the activity of going to a restaurant is a concurrent event for soccer training.

The change detecting section 54 acquires, as concurrent events, the two activities before and after the activity indicated by the read activity information, for example. As another example, the change detecting section 54 may acquire, as concurrent events, activities performed within one hour before and after the activity indicated by the read activity information.

If the information terminal control section 50 determines at step S714 that the activity has been repeated at least the predetermined number of times, the process moves to step S716. If the information terminal control section 50 determines that the activity has not been repeated at least the predetermined number of times, the process moves to step S724. At step S716, the information terminal control section 50 stores in the storage section 30, as new habit data, the combination of the activity and concurrent event. At step S718, the information providing section 40 performs the first related information providing process for providing information related to the activity accumulated as a habit.

On the other hand, if the change detecting section 54 determines at step S704 that the read activity information matches existing habit data, the process moves to step S720. At step S720, the information terminal control section 50 updates the performance frequency of the habit that the read activity information matches. Information indicating the performance frequency of each habit is associated with the corresponding existing habit data accumulated in the storage section 30, and the information terminal control section 50 updates the information indicating this performance frequency.

At step S722, the change detecting section 54 compares the updated performance frequency to a predetermined threshold value. The threshold value is set in advance for each habit and stored in the storage section 30. For example, a threshold value of every five days for a daily habit or a threshold value of every three months for a monthly habit may be set. The information terminal control section 50 may store threshold values input by the user via the manipulating section 12 in the storage section 30, or may update preset threshold values. If the frequency of a habit that was initially performed every day decreases or if the frequency of a habit that was initially performed every month increases to being performed every week, the information terminal control section 50 may update the threshold value described above for the corresponding habit.

At step S724, the information terminal control section 50 determines whether there is activity information that has yet to be processed. If it is determined that there is unprocessed activity information, the process moves to step S702 and this activity information is read. If it is determined that there is no unprocessed activity information, the process moves to step S726.

At step S726, the information terminal control section 50 compares the activity information read at step S702 to the habits of the user stored in the storage section 30, and extracts habits of the user for which there is no activity information. The following describes an example in which updating a blog and jogging are extracted as habits for which there is no activity information, and the frequency for each of these activities is once a week.

The information terminal control section 50 determines whether the update frequency of a habit that was not identified has decreased. The process flow ends if the update frequency of the identified habit is within a prescribed period, and the process proceeds to step S728 if the updated frequency of the identified habit has exceeded the prescribed period without there being an update. The frequency of the blog updating described above is once per week, and so a determination of "NO" is made at step S726 if only three days have passed since the previous update and a determination of "YES" is made at step S726 if one week has passed since the previous update. Furthermore, this example assumes that one week has passed since the previous performance of jogging.

At step S728, the information terminal control section 50 determines whether to suggest resuming the habit for which the update frequency has exceeded the prescribed period. The information terminal control section 50 makes a determination of "YES" at step S728 if the blog update described above has not been made for over a week. On the other hand, if jogging has not been performed for a week, the information terminal control section 50 extracts information such as weather, temperature, and humidity from the information extracting section 94 and acquires biometric information of the user from the biometric information acquiring section 16, for example. If a high temperature of 35°C or more has been continuing, if a low temperature near freezing has been continuing, or if the user is in poor health, the information terminal control section 50 makes a determination of "NO" at step S728. As described above, if it is determined based on the data from a body fat detecting scale that the weight and body fat of the user have increased, the information terminal control section 50 makes a determination of "YES" at step S728 to encourage the user to resume jogging. The information relating to temperature and humidity may be detected by providing a thermometer or humidity indicator in the environment acquiring section 20, instead of from the information extracting section 94.

At step S730, the information terminal control section 50 determines the date on which to suggest resumption of the habit to the user. Specifically, for the activity of updating the blog, the information terminal control section 50 schedules such a suggestion to be displayed in the display section 42 at a time to coincide with the day and time during which the user has updated the blog in the past. For resuming jogging, the information terminal control section 50 schedules the suggestion to be displayed in the display section 42 on the weekend. At that time, the information terminal control section 50 may schedule to reference and display the weather report, temperature, or humidity, for example. Since both of the habits in the above example are private activities, the information terminal control section 50 schedules the suggestion display described above to avoid times when it is determined from the output of the position detecting section 22 that the user is in a business area or when it is determined from the activity history of the user that the current time is when the user does business.

Prior to displaying the suggestion, the information terminal control section 50 may acquire biometric information of the user from the biometric information acquiring section 16 to confirm that the user is not feeling irritated, and may display the suggestion to resume the habit when it is determined that the user is relaxed.

The information terminal control section 50 counts the total time and the number of times a habit is performed and accumulates in the storage section 30, for each habit, information indicating whether the frequency of the habit is tending toward a decrease or an increase. In this case, the information terminal control section 50 stores this information in the storage section 30 in combination with information indicating whether resumption of the habit has been suggested. In this way, the information terminal control section 50 can check the frequency for each individual habit of the user and can also check the overall trend for the habits of the user, e.g. a decrease in the frequency of habits due to being busy at work or an abundance of personal time). Furthermore, if the frequency of a habit such as making monthly or yearly payments is decreasing, the information terminal control section 50 may display in the display section 42 notification that cost effectiveness is decreasing.

The above describes an example in which the habits are blogging and jogging, i.e. habits that can be performed by one person, but in the case of habits such as soccer that require a plurality of people, for example, a simple suggestion to the user to resume soccer is not effective. In this case, in the present embodiment, the information terminal control section 50 may extract Endo Shijuro, who is a soccer friend, from the person DB shown in Fig. 2, determine whether there has been contact with Endo Shijuro based on the environment acquiring section 20 and mail sending and receiving function described above, and suggest resuming soccer according to a trigger that there has been contact or that information concerning soccer is acquired by the event information acquiring section 96. In this case, the information terminal control section 50 may determine whether the number of times or total time spent contacting Endo Shijuro is on a decreasing trend or an increasing trend, and not make a suggestion for resumption if the contact with Endo Shijuro has been decreasing significantly.

The flow chart of Fig. 7 describes an example in which the determination as to whether to accumulate the activity information read from the storage section 30 as new habit data is based on the number of times the activity is repeated, but as another example, the biometric information of the user may be used as a basis for determination, in addition to the number of times the activity is repeated. For example, if there is little change in the heart rate of the user while performing an activity, it can be determined that the user has grown used to this activity and therefore there is a high probability that this activity is a habit. On the other hand, if there is a large change in the heart rate of the user while performing an activity, it can be determined that the user has not grown used to this activity, which possibly indicates that the user is nervous or excited, and therefore there is a high probability that this activity is not a habit. Therefore, when the number of repetitions is greater than or equal to a predetermined number of times and the change in the biometric information is less than a predetermined threshold value, the change detecting section 54 may determine that the activity information is a habit.

In addition to the number of repetitions of an activity, the voice of the user may also be used as a basis for determination. For example, if the user speaks the same word (e.g. the name of a famous person or sports star) many times within a certain period, it can be predicted that the user is interested in this word. In this case, the sound analyzing section 26 analyzes the text information resulting from a voice analysis performed on the voice of the user, and counts the number of time the user utters the word. If the number of utterances within a certain period exceeds a predetermined threshold value, the information terminal control section 50 registers the word analyzed by the sound analyzing section 26 in the storage section 30, as a word that the user is interested in. If the number of repetitions of an activity is greater than or equal to a predetermined number of times and referencing the words in which the user is interested registered in the storage section 30 shows a word corresponding to the activity registered in the storage section 30, the change detecting section 54 may determine that the activity information is a habit.

At step S710 and step S716 in the flow chart shown in Fig. 7, the schedule managing section 66 may reflect the habits indicated by the new habit data accumulated in the storage section 30 in the schedule information being managed. For example, when the habit of power walking near home from 20:00 to 21:00 every day is detected, the schedule managing section 66 adds the activity of power walking near home from 20:00 to 21:00 every day to the schedule information stored in the storage section 30.

The flow chart shown in Fig. 7 describes an example in which the information terminal 10 performs the process flow at 12:00 every night, but the information terminal 10 may instead perform this process flow every hour. Furthermore, this process flow may be performed every time the information terminal control section 50 gathers activity information.

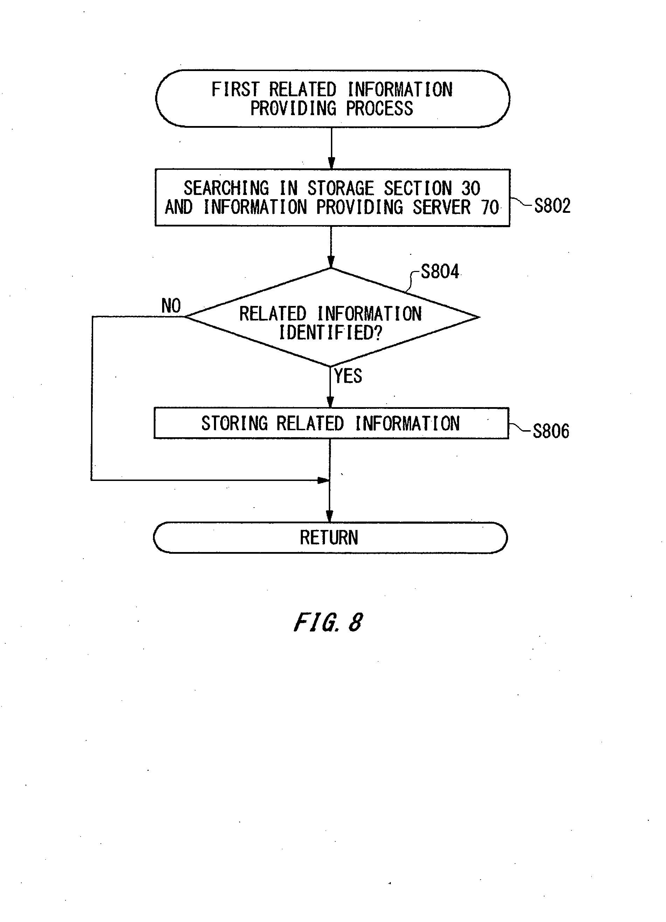

Fig. 8 is a flow chart showing a specific example of the first related information providing process at steps S712 and S718. At step S802, the information terminal control section 50 searches the information providing server 70 and the storage section 30 for information relating to an activity. The information terminal control section 50 searches the storage section 30 and the information providing server 70 using, as search conditions, the activity information identified at step S702. For example, when the activity information is "power walking near home," this matches the interest of Ito Jiro registered in the person DB 32 of the storage section 30. Therefore, the information terminal control section 50 transmits a control signal for scheduling a display in the display section 42 of Ito Jiro's name or image. At this time, the information terminal control section 50 has determined whether the activity information is private or non-private according to step S608 in the flow chart of Fig. 6, and therefore the information terminal control section 50 sets the display state in the display section 42 based on this determination result.

In the case of power walking, the information terminal control section 50 can determine that the activity is private based on the date and time detected by the time detecting section 21, the position detected by the position detecting section 22, and the image captured by the image capturing section 23, and therefore the information terminal control section 50 schedules the display for a private time. The display section 42 performs the display according to the received control signal for scheduling the display. Furthermore, when the audio output section 44 is used for voice guidance, the information terminal control section 50 may increase the volume of the audio output section 44 to be louder in private situations than in non-private situations.

For the activity information, if a change in the same activity information in the past is detected and this activity information has been registered as a habit, the information terminal control section 50 may acquire the past activity information as a search result. For example, for the activity information "power walking near home," if power walking near home has been set as a habit in the past, then this past activity information is a match. Therefore, the information terminal control section 50 transmits a control signal for scheduling the display section 42 to display the walking speed when power walking near home in the past and the time period over which the habit of power walking near home continued, for example. For a certain habit, by providing information concerning the same habit performed in the past, the user can be reminded of information that may have been forgotten.

When searching the information providing server 70, the information terminal control section 50 searches a related information database stored in the storage section 80 of the information providing server 70. In the related information database, for each habit, a webpage relating to the habit and information of users performing the habit are registered. For example, when searching the related information database for the activity of power walking near home, the information terminal control section 50 can acquire information such as a page selling power walking shoes and the walking speed and performance frequency of people who have the habit of power walking, for example. The present embodiment describes an example in which both the storage section 30 and the information providing server 70 are searched, but the target of the search may be just one of these.

At step S804, the information terminal control section 50 determines whether related information has been identified by the search at step S802. If related information was identified, the process moves to step S806, and if related information was not identified, the first related information providing process is ended. At step S806, the information terminal control section 50 stores the identified related information in the storage section 30.

The flow charts shown in Figs. 7 and 8 show examples in which the related information is provided for all of the new habit data accumulated in the storage section 30, but instead the related information may be provided when predetermined conditions are fulfilled. These predetermined conditions may be conditions for determining whether the user is interested in the detected habit. For example, the information terminal control section 50 may set, as the conditions, whether the user has searched for information relating to the activity in the past. Specifically, if the user has performed a search with "power walking" as a keyword, the information terminal control section 50 may transmit a control signal to the information providing section 40 to provide information relating to power walking. Upon receiving the control signal from the information terminal control section 50, the information providing section 40 provides the information relating to power walking through a display or audio output. By providing related information when a user has performed a search in the past, the related information can be provided for activities in which the user is predicted to have an interest.

The flow charts shown in Figs. 7 and 8 show an example in which the information terminal 10 detects a habit from the activity information and displays information relating to the detected habit, but at least a portion of a process other than display may be performed by the information providing server 70. For example, the information providing server 70 may receive in advance activity information of the user from the information terminal 10, and store this activity information in the storage section 80. Next, a change is detected in the received activity information and the habit is detected according to the frequency of the activity corresponding to the detected change. The information relating to the detected habit is then extracted from the storage section 80 and transmitted to the information terminal 10.

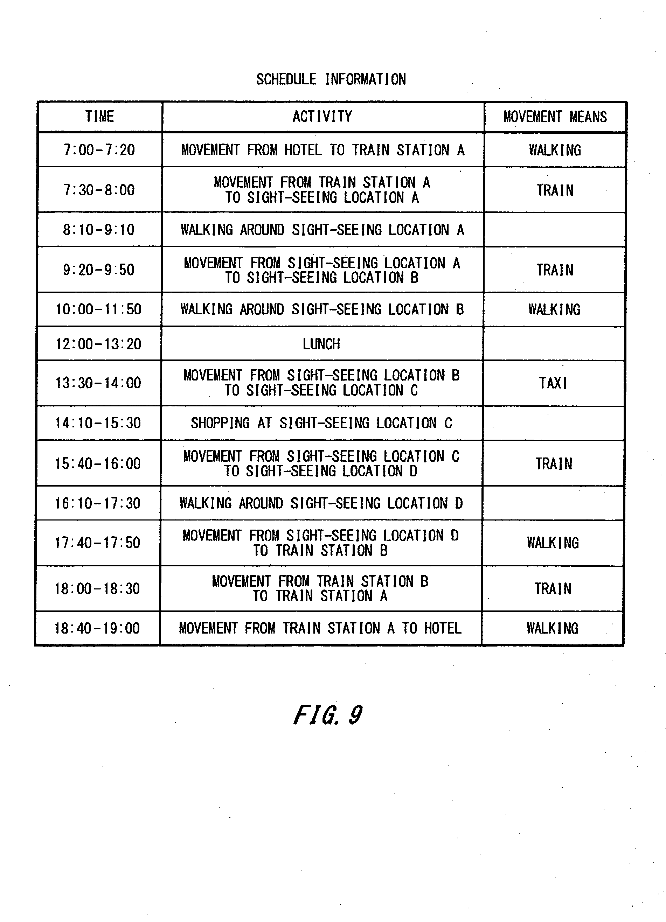

Fig. 9 shows an example of schedule information managed by the schedule managing section 66 according to a second embodiment. A time span from a start time to an end time, an activity planned to be performed during the time span, and a movement means to be used when performing the activity are registered in association with the schedule information. Combinations of time span, activity, and movement means are listed in temporal order from top to bottom. The following describes an example of a schedule input in advance by the user via the manipulating section 12 when the user visits a new region. For example, from 7:00 to 7:20, a scheduled item of moving by foot from a hotel to train station A is registered. The end time of each activity is referred to as the "scheduled end." For example, in the schedule item of moving by foot from the hotel to trains station A, 7:20 is the scheduled end.

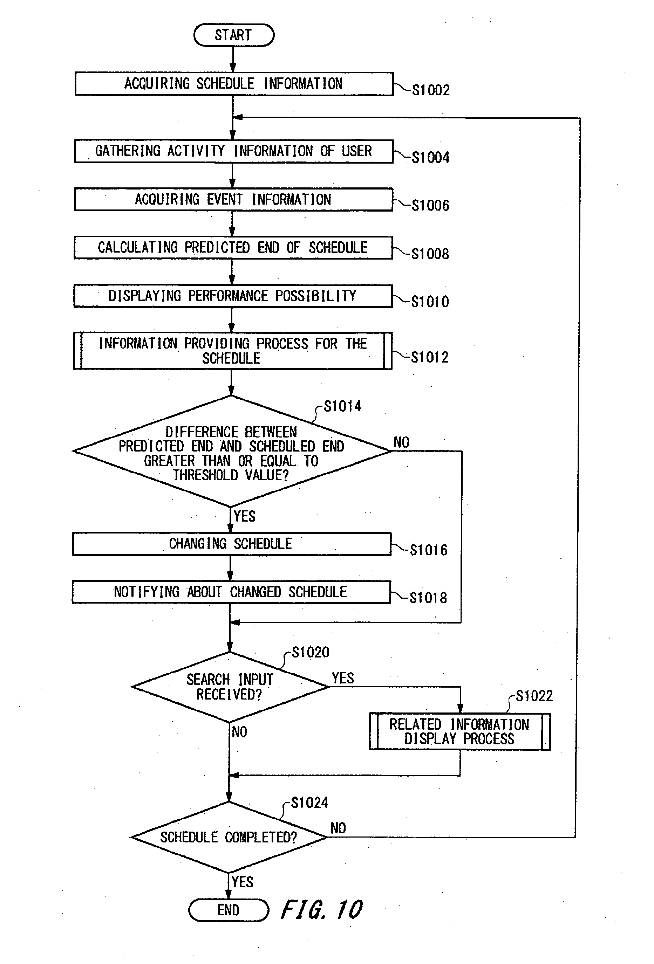

Fig. 10 is a flow chart showing a process using the activity information stored in the storage section 30 and the storage section 80, according to a second embodiment. The present embodiment describes an example in which activity information of the user performing activities according to a schedule input in advance is gathered, and information useful for fulfilling the schedule is provided based on the gathered activity information. Furthermore, the present embodiment describes an example in which, from among the pieces of activity information of the user stored in the storage section 80, activity information corresponding to a search input by the user is provided to the user. The hardware configuration for the information terminal 10 and the information providing server 70 in the second embodiment may be the same as in the first embodiment.

First, at step S1002, the schedule managing section 66 acquires the schedule information stored in the storage section 30. Here, an example is described in which the schedule information shown in Fig. 9 is acquired. Next, at step S1004, the information terminal control section 50 gathers activity information of the user. The information terminal control section 50 may use the environment acquiring section 20, the biometric information acquiring section 16, and the like to gather, as the activity information of the user, position information, movement speed, images, sound, biometric information of the user, personal information, available money information, and luggage information.

The biometric information acquiring section 16 acquires biometric information indicating that the user is tired or that the user is rushed, for example. If the results of the sound recognition performed by the sound analyzing section 26 on the sounds emitted by the user and collected by the sound gathering section 25 indicate a number of sneezes, a sniffling sound, and a scratchy voice, for example, the biometric information acquiring section 16 acquires biometric information indicating poor health. Furthermore, based on the utterances of the user received from the sound analyzing section 26, if keywords registered in advance as keywords indicating poor health such as "headache" or "caught a cold" are detected, the biometric information acquiring section 16 acquires biometric information indicating poor health.

The information terminal control section 50 stores the personal information concerning the gender and age of the user in the storage section 30, based on input through the manipulating section 12 or information acquired by the environment acquiring section 20. The information terminal control section 50 may acquire information concerning companions as one type of personal information, and in the second embodiment, the father "Okada Goro" is identified by the information terminal control section 50 as a companion based on images and sound acquired by the image capturing section 23 and the sound gathering section 25.

The information terminal control section 50 may detect the movement means of the user based on the electronic transaction section 58 performing an electronic transaction with a vending machine through the IC chip of the communicating section 48. The information terminal control section 50 can acquire luggage information by referencing the purchase information acquired by the electronic transaction section 58 to identify purchased items. Furthermore, a weight sensor may be provided in advance in a shoe of the user, for example, and the information terminal control section 50 may acquire the output of the weight sensor to detect carried luggage by detecting change in the weight. In other words, when the output of the weight sensor in a shoe is received through the communicating section 48 and an increase in weight is detected, the information terminal control section 50 can determine that luggage with a weight equal to the increase is being held. At this time, the weight of the user may be confirmed from the wireless scale described further above. Furthermore, if an image of the user himself captured by a webcam arranged on the street as an environment sensor 98 is acquired from the webcam through the communicating section 48, for example, the image analyzing section 24 may acquire the luggage information by performing image recognition on the captured image.

At step S1006, the event information acquiring section 64 acquires event information, such as information that an event is to be held at sight-seeing point B relating to the schedule information, information concerning the venue for the event, and information concerning traffic to this venue, for example. The event information acquiring section 64 can acquire the event information by accessing, through the communicating section 48, a webpage providing information about the event at sight-seeing location B and a webpage providing traffic information.

At step S1008, the information terminal control section 50 acquires a predicted end for the scheduled item currently being performed, from the activity information gathered at step S1004. The information terminal control section 50 calculates, as the predicted end, the predicted time at which the scheduled item currently being performed will end. The predicted end can be calculated from the difference between the start time of the scheduled item and the start time acquired from the gathered activity information.

For example, for the scheduled item of "moving from sight-seeing point A to sight-seeing point B from 9:20 to 9:50," if the start time acquired from the activity information is 9:30, the information terminal control section 50 can calculate the predicted end to be 10:00 by adding the 10-minute difference in start time to the scheduled item end time of 9:50. Furthermore, the information terminal control section 50 may reflect information such as companion information and information concerning luggage held by the user in the predicted end. A detailed calculation of a predicted end that reflects information concerning luggage and companions is described further below.

At step S1010, the information terminal control section 50 compares the calculated predicted end to the scheduled end in the schedule information, and calculates a performance possibility indicating the possibility that the scheduled item can be performed as scheduled. If the calculated predicted end is no later than the scheduled end in the schedule information, the information terminal control section 50 determines the performance possibility to be 100%. If the calculated predicted end is later than the scheduled end in the schedule information, the information terminal control section 50 can calculate the performance possibility by adopting a calculation technique by which the possibility decreases as the calculated predicted end becomes later.

Here, an example is described in which the ratio between the time needed for a scheduled item and the difference between the predicted end and the end time of the scheduled item is calculated by comparing a first predetermined threshold value to a second threshold value that is greater than the first threshold value. The information terminal control section 50 determines a high performance possibility when the ratio between the time needed for a scheduled item and the difference between the predicted end and the end time of the scheduled item is less than the first threshold value. The performance probability is determined to be neither high nor low when this ratio is greater than the first threshold value and less than the second threshold value, and the performance probability is determined to be low when this ratio is greater than the second threshold value.

Here, an example is described in which the first threshold value is set to 0.1 and the second threshold value is set to 0.25. For the schedule information concerning lunch from 12:00 to 13:00, when a predicted end of 13:05 is calculated, the time needed for the scheduled item is 60 minutes and the difference between the predicted end and the end time of the scheduled item is 5 minutes. In this case, the calculated ratio is approximately 0.08, which is less than the first threshold value, and therefore the information terminal control section 50 determines that the performance possibility is high. For the schedule information concerning lunch from 12:00 to 13:00, when a predicted end of 13:20 is calculated, the time needed for the scheduled item is 60 minutes and the difference between the predicted end and the end time of the scheduled item is 20 minutes. In this case, the calculated ratio is approximately 0.33, which is greater than the second threshold value, and therefore the information terminal control section 50 determines that the performance possibility is low.

When the information terminal control section 50 determines the calculated performance possibility to be low, the display section 42 displays information indicating the low performance possibility. The display section 42 may display text indicating the low performance possibility, or may display an image indicating the low performance possibility. When the information terminal control section 50 determines the calculated performance possibility to be high, the display section 42 displays text indicating the high performance possibility or an image indicating the high performance possibility. Here, the image indicating the high performance possibility and the image indicating the low performance possibility are images that enable the user to recognize the performance possibility, and may be images showing an X when the performance possibility is low and an O when the performance possibility is high, or images showing a yellow signal when the performance possibility is low and showing a blue signal when the performance possibility is high, for example.

At step S1012, the information providing section 40 performs an information providing process for the scheduled item. If the predicted end of the scheduled item is later than the predicted end of the scheduled item, the information providing section 40 displays information indicating that the user should hurry or information suggesting a change to the schedule. The information providing section 40 provides information based on the progress state of the schedule according to the activity information.

At step S1014, the information terminal control section 50 compares the predicted end calculated at step S1008 to the predicted end in the schedule, and determines whether the difference between the predicted end and the scheduled end exceeds a predetermined threshold value. If the difference between the predicted end and the scheduled end is greater than or equal to the predetermined threshold value, the process moves to step S1016. If the difference is not greater than or equal to the threshold value, the process moves to step S1020.

At step S1016, the schedule changing section 68 changes the schedule, which is the schedule to be performed in the future managed by the schedule managing section 66, based on the difference between the predicted end and the scheduled end. For example, when the predetermined threshold value is 15 minutes and the predicted end for a scheduled item of arriving at train station A at 18:30 is 18:10, the schedule changing section 68 changes the predetermined route for moving from train station A to the hotel to be a longer route that includes a location recorded in advance in the storage section 30.

As a specific example of a preregistered location, there may be a location that is worth visiting, such as a place that is famous for cherry blossoms. The storage section 30 may store information of locations input in advance through the manipulating section 12, for example. As another example, the storage section 30 may store location information acquired through the communicating section 48 from a server providing recommended location information. The schedule changing section 68 may reference the location information stored in the storage section 80 through the communicating section 48. On the other hand, if the predicted end is later than the scheduled end, the schedule changing section 68 makes a change to shorten or delete scheduled items.

At step S1018, the information providing section 40 provides notification of the changed schedule. Along with the notification of the changed schedule, the information providing section 40 may also provide information for the changed schedule, in the same manner as at step S1012.

In the present embodiment, the information terminal 10 has a function to acquire activity information corresponding to a search input, from among the pieces of user activity information stored in the information providing server 70, and provide this activity information to the user. For example, when a search input is received stating a desire for activity information of people who have walked around sight-seeing location B, the information terminal 10 acquires from the information providing server 70 the activity information of people who have walked around sight-seeing location B in the past, and provides this activity information to the user. At step S1020, the information terminal control section 50 determines whether a search input from the user has been received.

Specifically, first, the information terminal control section 50 causes the display section 42 to display a screen awaiting reception of a search input, in response to instructions from the user via the manipulating section 12. The information terminal control section 50 may display this reception screen at any timing when instructions are received from the user, and the display is not limited to the timing of step S1020. The information terminal control section 50 receives, as the search input, input of an activity target indicating a user activity target. The display section 42 displays, as the reception screen, an input box into which the activity target is input.

The information terminal control section 50 may display walking, shopping, moving, and the like as activity target candidates in the display section 42. When walking is displayed as a candidate, the display section 42 displays an inquiry about the location for the walk, as a portion of the activity target. As another example, when shopping is displayed as a candidate, the display section 42 displays inquiries concerning the shopping location and the items to be bought while shopping. When moving is displayed as a candidate, the display section 42 displays inquiries concerning where the user is moving from and where the user is moving to. The user can input the activity target by selecting from among the displayed candidates.

When inquiries concerning a location of a walk, a location for shopping, and a start point and destination for moving are displayed, the information terminal control section 50 may display the names of locations shown in the current region of the information terminal 10 as candidates in the display section 42. Furthermore, in order to receive designation of a location, the display section 42 may display a map of the current region of the information terminal 10 that allows for selection. When the intended activity target is included among the candidates, the user responds to the inquiries by selecting the candidate. When the intended activity target is not included among the candidates, the user responds to the inquiries by providing direct input to the input box. The information terminal control section 50 stores the received search inputs in the storage section 30, in association with the time at which the search inputs were received, as detected by the time detecting section 21.

When the information terminal control section 50 determines at step S1020 that a search input has been received through the above process, the process moves to step S1022. At step S1022, the information terminal control section 50 performs a related information display process to display the related information including the activity information corresponding to the search input. A detailed description of the related information display process is provided further below. After the related information display process, the information terminal control section 50 proceeds to step S1024.

When the information terminal control section 50 determines at step S1020 that a search input has not been received, the process moves to step S1024. At step S1024, the information terminal control section 50 determines whether the schedule is complete, by comparing the activity information acquired at step S1004 to the schedule information. If the schedule is not completed, the process returns to step S1004. If the schedule is completed, the process ends.

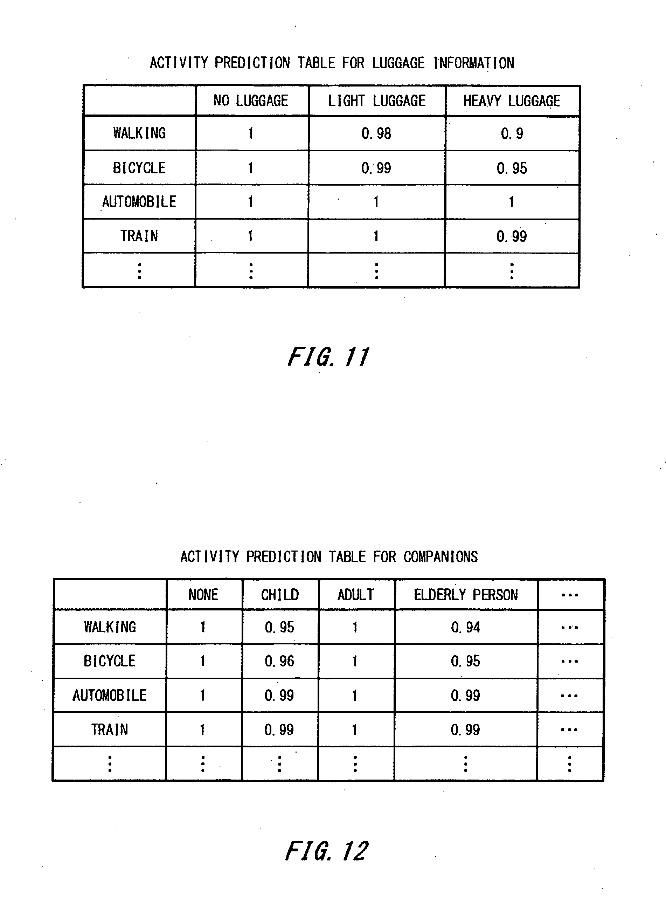

Fig. 11 shows an exemplary activity prediction table for luggage information, stored in the storage section 30. The activity prediction table is a table database in which is registered schedule progress coefficients applied to a matrix of movement means that can be used and activity restrictions of the user predicted for the activity information. The schedule progress coefficients have lower values when the amount of interference in the schedule progress is greater.

In the activity prediction table shown in Fig. 11, for the movement means of "walking," schedule progress coefficients of 1 when there is no luggage, 0.98 when there is light luggage, and 0.90 when there is heavy luggage are registered as activity restrictions. When calculating the predicted end of the scheduled item currently being performed, the information terminal control section 50 can use the schedule progress coefficients.

The information terminal control section 50 calculates the predicted end by calculating the extra time required, which is obtained as the product of the scheduled time and a value obtained by subtracting the schedule progress coefficient from 1. For example, for the scheduled item of moving by foot from the hotel to train station A, which requires 20 minutes, if luggage information indicating that the user is carrying heavy luggage is gathered as the activity information, an extra time of 2 minutes is calculated as the product of 20 minutes and the value 0.1 obtained by subtracting 0.9 from 1. The information terminal control section 50 calculates the predicted end time as a time 2 minutes after the scheduled time. By referencing the activity prediction table in this way, the accuracy of the predicted end of scheduled items can be improved.

The information terminal control section 50 may acquire the predicted end by referencing activity information of people other than the user stored in the storage section 80 of the information providing server 70. For example, when the schedule information indicates moving from sight-seeing location A to sight-seeing location B, the information terminal control section 50 may acquire the time spent from the activity information of other people who have moved from sight-seeing location A to sight-seeing location B, and use this time to se the predicted end.

Fig. 12 shows an exemplary activity prediction table for companions. In the activity prediction table shown in Fig. 12, low schedule progress coefficients are set when the companion is elderly or a child. For example, for the movement means of walking, the schedule progress coefficient is 0.95 when the companion is a child and the schedule progress coefficient is 1 when the companion is an adult, thereby enabling the predicted end of the scheduled item to reflect the fact that walking with a child causes more impedance to the progress of the schedule than walking with an adult. In the second embodiment, since the companion is Okada Goro, as described above, the information terminal control section 50 sets 0.94 as the schedule progress coefficient. More detailed registration data may be set, such as registering companions according to each age and registering a plurality of companions.

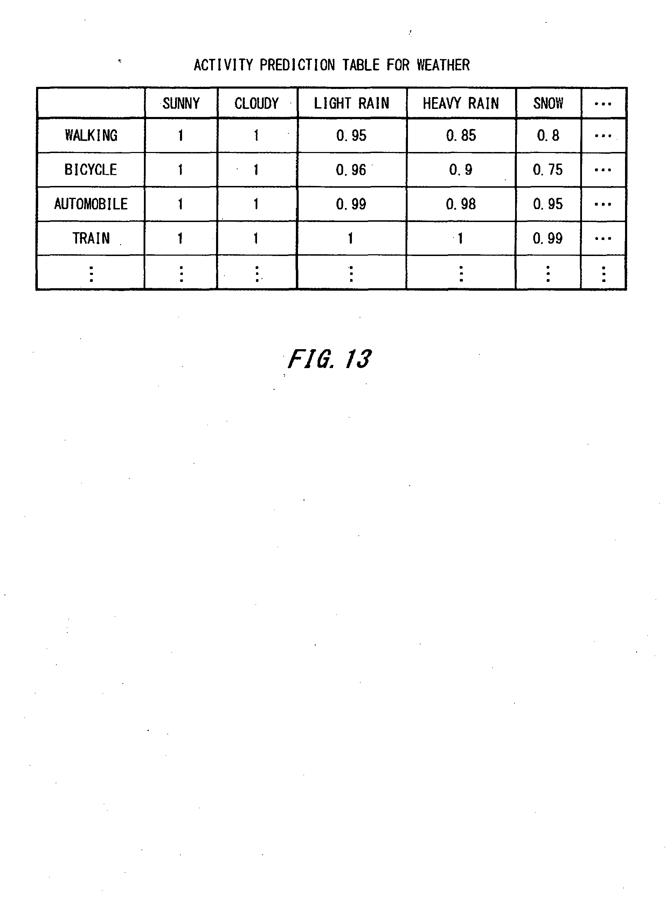

Fig. 13 shows an exemplary activity prediction table for weather. In the activity prediction table shown in Fig. 13, lower schedule progress coefficient values are registered for worse weather. For example, for the movement means of walking, the schedule progress coefficient is 0.95 when the weather is light rain and the schedule progress coefficient is 0.85 when the weather is heavy rain, thereby enabling the predicted end of the scheduled item to reflect the fact that walking in heavy rain causes more impedance to the progress of the schedule than sunny weather. The information terminal control section 50 may acquire the weather information by referencing a webpage that provides weather information, via the communicating section 48.

The storage section 30 may also include an activity prediction table for sleep time, as an activity prediction table for biometric information. The biometric information acquiring section 16 can acquire sleep time information of the user by communicating with a sleep sensor for analyzing sleep of the user. As another example, blinking of the user can be detected, a correspondence relationship between blinking and sleep time can be obtained verifiably, and a table showing this correspondence relationship can be stored in the storage section 30 in advance. In this activity prediction table for sleep time, lower schedule progress coefficients are set for shorter sleep times. Furthermore, the storage section 30 may store activity prediction tables corresponding to the tone of voice of the user or the time that has passed from when the user awoke.

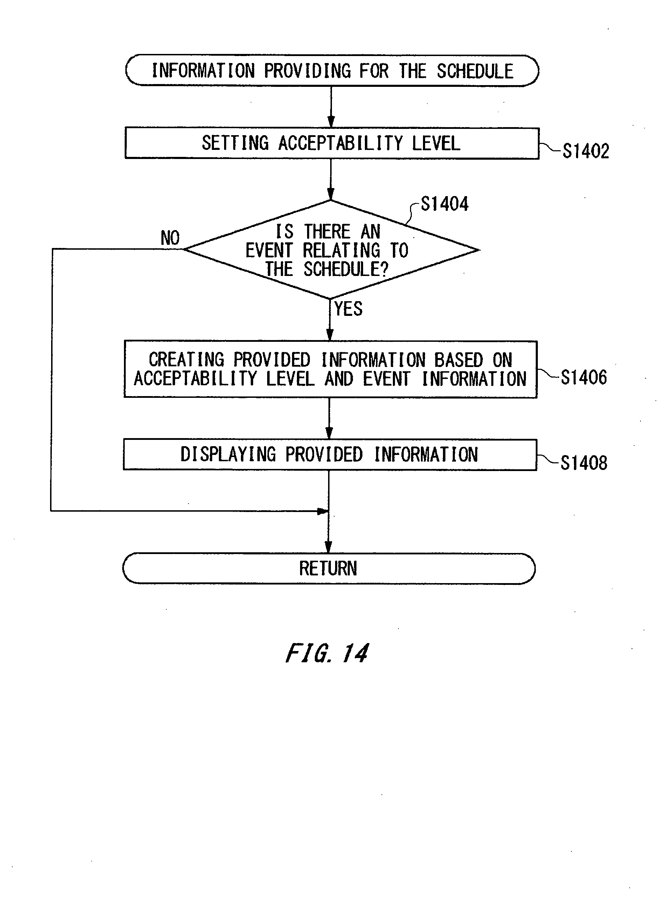

Fig. 14 is a flow chart showing a specific information providing process for the schedule of step S1012. First, at step S1402, the information terminal control section 50 acquires an acceptability level for change in the schedule. The acceptability level for change in the schedule is a lower value when the effect on later scheduled items is larger as the result of the change to the schedule.

For example, concerning a case of being late for a flight and a case of being late for a train, being late for the flight has a greater impact on scheduled items, and therefore the information terminal control section 50 sets a lower acceptability level for scheduled items before getting on the flight. Furthermore, even among trains, between a train in an urban area and a train in a rural area where the trains run less frequently, missing the rural train has a greater impact on later scheduled items. Therefore, the information terminal control section 50 sets a lower acceptability level for the scheduled item of moving to a rural train station than for the scheduled item of moving to an urban train station. This acceptability level may be set by input through the manipulating section 12.

The set acceptability levels are stored in the storage section 30 or the storage section 80. The information terminal control section 50 searches for an alternate means when the schedule is changed, and sets a lower acceptability level when there is more excess time. For example, when moving from sight-seeing location A to sight-seeing location B, the information terminal control section 50 calculates the difference in arrival time at sight-seeing location N between the train that is to be ridden according to the schedule and the next train after this scheduled train, and sets a lower acceptability level when this calculated difference is greater.

At step S1404, the information terminal control section 50 determines whether there is an event relating to the schedule within the event information acquired at step S1006. If there is event information for an even occurring at a location near a location included in the movement route in the schedule, the information terminal control section 50 determines that there is a related event. For example, for the scheduled item "moving from sight-seeing location B to sight-seeing location C," if there is information of a traffic jam on the movement route from sight-seeing location B to sight-seeing location C, the information terminal control section 50 determines that there is a related event. If there is determined to be an event relating to the schedule at step S1404, the process proceeds to step S1406, and if there is determined to be no related event, this process flow is ended.

At step S1406, the information terminal control section 50 creates provided information to be provided to the information providing section 40, based on the event information acquired at step S1006 and the acceptability level acquired at step S1402. Specifically, the information terminal control section 50 creates provided information recommending that the schedule proceed as planned for a scheduled item with a low acceptability level, and the provided information may be image information warning that it is necessary to hurry. As another example, the information terminal control section 50 may create, as the provided information, text data indicating that the user is behind schedule, for a scheduled item having a high acceptability level.

The information terminal control section 50 creates, as the provided information to be provided to the information providing section 40, event information relating to the schedule. For example, for the scheduled item of moving by taxi from sight-seeing location B to sight-seeing location C, if event information of a traffic jam in the movement route is acquired, the information terminal control section 50 creates, as the provided information, text indicating that there is a traffic jam in the movement path.

At step S1406 in the flow chart shown in Fig. 14, when creating the provided information, the provided information may be created together with movement of the user. For example, when the user is moving from sight-seeing location A to sight-seeing location B and the movement speed of the user is lower than the average movement speed of the user, the information terminal control section 50 creates provided information that recommends a path that would lessen the burden on the user, such as a path without stairs or a path without hills on the movement route. The information terminal control section 50 displays the information created at step S1406 in the display section 42 at step S1408, and this process flow is then ended.

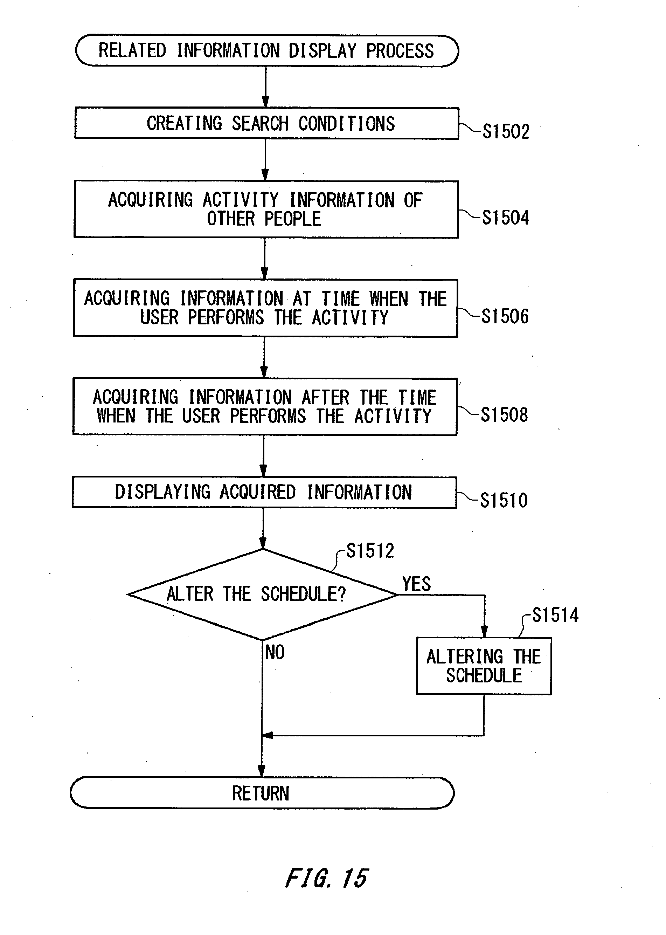

Fig. 15 is a flow chart showing a specific related information display process of step S1022. At step S1502, the information terminal control section 50 creates search conditions based on the activity information of the user himself gathered at step S1004 and the search inputs received at step S1020. Furthermore, the information terminal control section 50 may add at least one of the available money information, personal information, and biometric information of the user to the search conditions. Here, it is assumed that a search is made for information concerning sight-seeing location B at 9:30 a.m. while the user is moving from sight-seeing location A to sight-seeing location B, and personal information indicating that a 50-year old father is present and that the available money for these two people is approximately 20,000 Yen is input.

At step S1504, the information terminal control section 50 acquires the activity information corresponding to the search input from the activity information of a plurality of users (other users) stored in the storage section 80. The information providing server control section 90 searches, among past activity information already stored in the storage section 80, for activity information of a time when a 50-year old father and son visited sight-seeing location B and activity information for walking around sight-seeing location B with a budget of approximately 20,000 Yen.

Here, the information providing server control section 90 acquires information concerning a walking route for sight-seeing location B (e.g. a walking route from point A to point D to point B), souvenir information, and lunch restaurant information, for example, as the received search conditions. Since the presence of the 50-year old father is detected as the personal information, the information providing server control section 90 may provide route guidance by choosing a path that is flat without significant slopes or a path that does not include stairs or bridges, for example, when moving by foot. In the above example, the information providing server control section 90 performs the search based on the activity information of other people, but if the user has visited sight-seeing location B before, the activity information of the user himself may be searched for.

At step S1506, the information terminal control section 50 acquires information for the point in time when the user will perform the activity. The information terminal control section 50 acquires information (weather information, traffic information, crowding condition) relating to sight-seeing location B at 9:30 a.m. from the information providing server control section 90.

The information terminal control section 50 acquires the information based on the characteristics of the date and time associated with the time when the user performs the activity. For example, the information terminal control section 50 may reference registered data in which is registered dates and times when there is a statistically large amount of traffic. The information terminal control section 50 determines whether the date and time associated with the time when the user performs the activity corresponds to a date and time when there is a statistically large amount of traffic. Dates and times when there is a statistically large amount of traffic may include weekends, the end of the month, or holidays, for example.

At step S1508, the information terminal control section 50 acquires information for times after the point in time when the user performs the activity. Since the arrival time of the user at sight-seeing location B is around 10:00, the information terminal control section 50 acquires event information for sight-seeing location B after 10:00 by using the information providing server control section 90, and also acquires predicted crowding information for sight-seeing location B after 10:00, traffic information when moving to sight-seeing location C, which is scheduled to be visited after sign-seeing location B, and a weather report for the area around sight-seeing location C from 2:00 onward.

At step S1510, the information acquired at steps S1504, S1506, and S1508 by the information terminal control section 50 is displayed in the display section 42. At this time, the information terminal control section 50 may prioritize the display of information relating to a change of the user's schedule. For example, if a traffic jam is anticipated during movement by taxi from sight-seeing location B to sight-seeing location C, the information terminal control section 50 may display that a traffic jam is expected in the display section 42. As another example, if rain is anticipated from the evening near train station A according to the weather report and the user has sufficient available money, the information terminal control section 50 displays in the display section 42 the weather report and the expected cost of a taxi ride from station A to the hotel.

On the other hand, when the display section 42 displays information concerning an event held at point D within sight-seeing location B, for example, the display section 42 may display an icon, character string, or the like on a map of sight-seeing location B indicating that an event is being held at point D. As another example, when showing information of another event planned to be held one hour later at point E within sight-seeing location B, the display section 42 may display an icon, character string, or the like on a map of sight-seeing location B indicating the an event is to be held one hour later at point E.

Here, if the movement speed of the user moving by foot from the hotel to train station A is lower than the average walking speed of the user by at least a predetermined threshold amount, the information terminal control section 50 may determine that there is a possibility that the user is tired or carrying heavy luggage, and then, if the movement distance to the point at which the event is being held is greater than a predetermined distance, the information terminal control section 50 need not display information for this event. On the other hand, if the movement speed of the user is higher than the average walking speed, the information terminal control section 50 may display information for an event even if the event is farther than a predetermined distance.

At step S1512, the information terminal control section 50 asks the user whether the schedule should be altered, based on the information shown in the display section 42 at step S1510. For example, the information terminal control section 50 may display questions in the display section 42 concerning whether to make the departure time to sight-seeing location C earlier or whether to take a taxi from train station A to the hotel, as described above, and obeys the input of the user through the manipulating section 12. When the input of the user from the manipulating section 12 indicates a change in the schedule, the information terminal control section 50 proceeds to step S1514.

At step S 1514, the information terminal control section 50 alters the schedule based on the input of the user, and displays the altered schedule in the display section 42. Furthermore, if there is an unexpected cost, e.g. the cost of a taxi from train station A to the hotel, the information terminal control section 50 displays in the display section 42 the amount of available money that can be used freely. In the above description the information was provided to the user through the display section 42, but the audio output section 44 may be used instead to provide voice guidance.

When creating the search conditions at step S1502, the information terminal control section 50 may receive property information indicating a property of the activity as a search input, and add this property information to the search conditions. This property information may include cost priority or time priority, for example. If cost priority is added to the search conditions, at step S1504, the information providing server control section 90 extracts activity information associated with cost priority from among the plurality of types of activity information classified according to different properties.

The association of the property information with activity information that has been accumulated is performed at the time when the activity information is gathered. For example, the information terminal control section 50 may display in the display section 42 a question posed to the user in advance as to whether an activity should prioritize cost or prioritize time. By receiving the input of the user in the display, the information terminal control section 50 acquires the property information and associates the property information with the activity information. The information terminal control section 50 may display in the display section 42 a question posed to the user about the property information after the activity has ended. The information terminal control section 50 stores the acquired property information in the storage section 30 in association with the acquired activity information. The property information and activity information stored in the storage section 30 are stored in the storage section 80 via the communicating section 48.

The flow chart of Fig. 15 describes an example focusing on providing information relating to sight-seeing, but instead information relating to work progress may be provided in a business setting.

If target information such as passing the bar exam is received as a search input, the information terminal control section 50 may provide the activity information of other users who input passing the bar exam as target information. Yet further, the information terminal control section 50 may continuously acquire the target information from when the search input is input, and periodically provide this information. By providing the activity information of people having the same target, which may be information concerning books if the person purchased a book or information concerning a prep school if the person began attending a prep school, the user can be provided with information that is useful in achieving the target.

When target information such as passing the bar exam is received as a search input, the information terminal control section 50 may acquire the activity information of people who have achieved the target, i.e. people who have received their bar credentials, and provide this activity information to the information providing section 40. By registering the certifications held by users in advance in the storage section 80, the information providing server 70 can identify users who have their bar credentials. By providing activity information of people who have achieved their targets, information in which the user has an interest can be provided.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### List of Reference Numerals

10: information terminal, 12: manipulating section, 14: acceleration detecting section, 16: biometric information acquiring section, 20: environment acquiring section, 21: time detecting section, 22: position detecting section, 23: image capturing section, 24: image analyzing section, 25: sound gathering section, 26: sound analyzing section, 30: storage section, 32: person DB, 34: activity DB, 40: information providing section, 42: display section, 44: audio output section, 48: communicating section, 50: information terminal control section, 52: activity identifying section, 54: change detecting section, 56: movement detecting section, 58: electronic transaction section, 62: available money managing section, 64: event information acquiring section, 66: schedule managing section, 68: schedule changing section, 70: information providing server, 78: communicating section, 80: storage section, 90: information providing server control section, 94: information extracting section, 96: event information acquiring section, 98: environment sensor, 100: personal assistance system

## Claims

1. An information terminal comprising:
an activity identifying section (52) that gathers activity information of a user;
a first detecting section (54) that detects change in the gathered activity information;
***characterized by***
a second detecting section (56) that detects movement of the user including a speed of the user,
an information providing section (40) that, based on frequency of an activity corresponding to the detected change, provides information relating to the activity, the information providing section (40) providing the information based on the detected movement, wherein
the activity identifying section (52) identifies an activity causing the speed of the user to be different from a predetermined movement speed when the speed of the user is different from the predetermined movement speed.

2. The information terminal according to claim 1, wherein when the activity identifying section gathers (52), as the activity information, searches relating to the information made by the user in the past, the information providing section (40) provides the information.

3. The information terminal according to claim 1, further comprising a position detector (22) that detects a position of die user, wherein the activity identifying section (52) identifies the activity causing the speed of the user to be different from the predetermined movement speed using the position of the user.

4. The information terminal according to claim 1, wherein the activity identifying section (52) identifies the activity causing the speed of the user to be different from the predetermined movement speed by determining whether the position of the user is within a prescribed range of a location recorded in advance.

5. The information terminal according to any one of claims 1 to 4, further comprising a biometric information acquiring section (16) that acquires biometric information of the user, wherein the activity identifying section (52) identifies the activity causing the speed of the user to be different from the predetermined movement speed using the biometric information of the user.

6. The information terminal according to any one of claims 1 to 5, wherein the information providing section (40) provides at least one of a person or a product, the person being interested in the activity causing the speed of the user to be different from the predetermined movement speed, and the product being useful for the activity causing the speed of the user to be different from the predetermined movement speed.

7. The information terminal according to claim 6, wherein the information providing section (40) provides information for a scheduled item.

8. The information terminal according to any one of claims 1 to 7, wherein the activity identifying section (52) identifies the activity causing the speed of the user to be different from the predetermined movement speed as one of power walking and jogging.

9. The information terminal according to any one of claims 1 to 8, wherein the activity identifying section (52) identifies an activity causing the speed to be different from a normal walking speed as the activity causing the speed of the user to be different from the predetermined movement speed.

10. The information terminal according to any one of claims 1 to 9, wherein the information terminal is adapted to be carried by a human.

## Patentansprüche

1. Informationsendgerät, umfassend:
einen Aktivitäts-Identifizierungsabschnitt (52), der Aktivitätsinformation eines Benutzers sammelt;
einen ersten Erfassungsabschnitt (54), der eine Änderung in der gesammelten Aktivitätsinformation erfasst;
**gekennzeichnet durch**
einen zweiten Erfassungsabschnitt (56), der eine Bewegung des Benutzers, enthaltend eine Geschwindigkeit des Benutzers, erfasst,
einen Informations-Bereitstellungsabschnitt (40), der, basierend auf einer Frequenz einer Aktivität entsprechend der erfassten Änderung, eine Information betreffend die Aktivität bereitstellt, wobei der Informations-Bereitstellungsabschnitt (40) die Information basierend auf der erfassten Bewegung bereitstellt, wobei
der Aktivitäts-Identifizierungsabschnitt (52) eine Aktivität identifiziert, die verursacht, dass sich die Geschwindigkeit des Benutzers von einer vorbestimmten Bewegungsgeschwindigkeit unterscheidet, wenn die Geschwindigkeit des Benutzers sich von der vorbestimmten Bewegungsgeschwindigkeit unterscheidet.

2. Informationsendgerät nach Anspruch 1, wobei, wenn der Aktivitäts-Identifizierungsabschnitt (52), als die Aktivitätsinformation, Suchen betreffend die Information, die der Benutzer in der Vergangenheit gemacht hat, sammelt, der Informations-Bereitstellungsabschnitt (40) die Information bereitstellt.

3. Informationsendgerät nach Anspruch 1, das weiterhin einen Positionsdetektor (22) umfasst, der eine Position des Benutzers erfasst, wobei der Aktivitäts-Identifizierungsabschnitt (52) die Aktivität, die verursacht, dass die Geschwindigkeit des Benutzers sich von der vorbestimmten Bewegungsgeschwindigkeit unterscheidet, unter Verwendung der Position des Benutzers identifiziert.

4. Informationsendgerät nach Anspruch 1, wobei der Aktivitäts-Identifizierungsabschnitt (52) die Aktivität identifiziert, die verursacht, dass die Geschwindigkeit des Benutzers sich von der vorbestimmten Bewegungsgeschwindigkeit unterscheidet, durch Bestimmen, ob die Position des Benutzers innerhalb eines vorgeschriebenen Bereichs eines im Voraus aufgezeichneten Ortes ist.

5. Informationsendgerät nach einem der Ansprüche 1-4, das weiterhin einen biometrischen Informations-Erfassungsabschnitt (16) umfasst, der biometrische Information des Benutzers erfasst, wobei der Aktivitäts-Identifizierungsabschnitt (52) die Aktivität, die verursacht, dass die Geschwindigkeit des Benutzers sich von der vorbestimmten Bewegungsgeschwindigkeit unterscheidet, unter Verwendung der biometrischen Information des Benutzers identifiziert.

6. Informationsendgerät nach einem der Ansprüche 1-5, wobei der Informations-Bereitstellungsabschnitt (40) eine Position und/oder ein Produkt bereitstellt, wobei die Person an der Aktivität interessiert ist, die verursacht, dass die Geschwindigkeit des Benutzers sich von der vorbestimmten Bewegungsgeschwindigkeit unterscheidet, und das Produkt für die Aktivität verwendbar ist, die verursacht, dass die Geschwindigkeit des Benutzers sich von der vorbestimmten Bewegungsgeschwindigkeit unterscheidet.

7. Informationsendgerät nach Anspruch 6, wobei der Informations-Bereitstellungsabschnitt (40) eine Information für ein geplantes Element bereitstellt.

8. Informationsendgerät nach einem der Ansprüche 1-7, wobei der Aktivitäts-Identifizierungsabschnitt (52) die Aktivität, die verursacht, dass die Geschwindigkeit des Benutzers sich von der vorbestimmten Bewegungsgeschwindigkeit unterscheidet, als eines von Powerwalking oder Joggen identifiziert.

9. Informationsendgerät nach einem der Ansprüche 1-8, wobei der Aktivitäts-Identifizierungsabschnitt (52) eine Aktivität, die verursacht, dass die Geschwindigkeit sich von einer normalen Gehgeschwindigkeit unterscheidet, als die Aktivität identifiziert, die verursacht, dass die Geschwindigkeit des Benutzers sich von der vorbestimmten Bewegungsgeschwindigkeit unterscheidet.

10. Informationsendgerät nach einem der Ansprüche 1-9, wobei das Informationsendgerät ausgebildet ist, von einem Menschen getragen zu werden.

## Revendications

1. Terminal d'informations comprenant :
une section d'identification d'activité (52) qui collecte des informations d'activité d'un utilisateur ;
une première section de détection (54) qui détecte un changement dans les informations d'activité collectées ;
**caractérisé par**
une deuxième section de détection (56) qui détecte un déplacement de l'utilisateur y compris une vitesse de l'utilisateur,
une section de fourniture d'informations (40) qui, sur la base d'une fréquence d'une activité correspondant au changement détecté, fournit des informations relatives à l'activité, la section de fourniture d'informations (40) fournissant les informations sur la base du déplacement détecté, où
la section d'identification d'activité (52) identifie une activité amenant la vitesse de l'utilisateur à être différente d'une vitesse de déplacement prédéterminée lorsque la vitesse de l'utilisateur est différente de la vitesse de déplacement prédéterminée.

2. Terminal d'informations selon la revendication 1, dans lequel, lorsque la section d'identification d'activité (52) collecte, comme étant les informations d'activité, des recherches relatives aux informations faites par l'utilisateur dans le passé, la section de fourniture d'informations (40) fournit les informations.

3. Terminal d'informations selon la revendication 1, comprenant en outre un détecteur de position (22) qui détecte une position de l'utilisateur, dans lequel la section d'identification d'activité (52) identifie l'activité amenant la vitesse de l'utilisateur à être différente de la vitesse de déplacement prédéterminée en utilisant la position de l'utilisateur.

4. Terminal d'informations selon la revendication 1, dans lequel la section d'identification d'activité (52) identifie l'activité amenant la vitesse de l'utilisateur à être différente de la vitesse de déplacement prédéterminée en déterminant si la position de l'utilisateur est comprise dans une plage prescrite d'un emplacement enregistré à l'avance.

5. Terminal d'informations selon l'une quelconque des revendications 1 à 4, comprenant en outre une section d'acquisition d'informations biométriques (16) qui acquiert des informations biométriques de l'utilisateur, dans lequel la section d'identification d'activité (52) identifie l'activité amenant la vitesse de l'utilisateur à être différente de la vitesse de déplacement prédéterminée en utilisant les informations biométriques de l'utilisateur.

6. Terminal d'informations selon l'une quelconque des revendications 1 à 5, dans lequel la section de fourniture d'informations (40) fournit au moins l'un(e) parmi une personne ou un produit, la personne étant intéressée par l'activité amenant la vitesse de l'utilisateur à être différente de la vitesse de déplacement prédéterminée, et le produit étant utile pour l'activité amenant la vitesse de l'utilisateur à être différente de la vitesse de déplacement prédéterminée.

7. Terminal d'informations selon la revendication 6, dans lequel la section de fourniture d'informations (40) fournit des informations pour un élément planifié.

8. Terminal d'informations selon l'une quelconque des revendications 1 à 7, dans lequel la section d'identification d'activité (52) identifie l'activité amenant la vitesse de l'utilisateur à être différente de la vitesse de déplacement prédéterminée en tant qu'une activité parmi la marche rapide et le jogging.

9. Terminal d'informations selon l'une quelconque des revendications 1 à 8, dans lequel la section d'identification d'activité (52) identifie une activité amenant la vitesse à être différente d'une vitesse de marche normale comme étant l'activité amenant la vitesse de l'utilisateur à être différente de la vitesse de déplacement prédéterminée.

10. Terminal d'informations selon l'une quelconque des revendications 1 à 9, dans lequel le terminal d'informations est adapté pour être porté par un être humain.
